# EUROPEAN PATENT APPLICATION

(11) **EP 3 263 031 A1**
(43) Date of publication of application: **03.01.2018**
(21) Application number: 17173680.4
(22) Date of filing: 31.05.2017
(51) Int. Cl.: A61B 5/11, A61B 5/00

(54) **SITUATION IDENTIFICATION METHOD, SITUATION IDENTIFICATION DEVICE, AND SITUATION IDENTIFICATION PROGRAM**

(30) Priority: 29.06.2016 JP 2016129271
(71) Applicant: Fujitsu Limited, Kawasaki-shi, Kanagawa 211-8588 (JP)
(72) Inventor: Okada, Yasutaka, Kawasaki-shi, Kanagawa 211-8588 (JP); Murashita, Kimitaka, Kawasaki-shi, Kanagawa 211-8588 (JP)
(74) Representative: Hoffmann Eitle

(57) **Abstract**

A situation identification method includes acquiring a plurality of images; identifying, for each of the plurality of images, a first area including a bed area where a place to sleep appears in an image, and a second area where an area in a predetermined range around the place to sleep appears in the image; detecting a state of a subject to be monitored for each of the plurality of images based on a result of detection of a head area indicating an area of a head of the subject in the first area and a result of detection of a living object in the second area; when the state of the subject changes from a first state to a second state, identifying a situation of the subject based on a combination of the first state and the second state; and outputting information that indicates the identified situation.

## Description

### TECHNICAL FIELD

The embodiments discussed herein are related to a situation identification method, a situation identification device, and a situation identification program.

### BACKGROUND ART

Some monitoring systems are known that monitor activities of patients lying on beds, care receivers, and the like in a medical institution, a nursing home or the like by using a camera instead of by a healthcare professional such as a nurse, a care worker, the activities including awaking up or leaving their beds and the manner how the patients or care receivers are lying on their beds (see, for instance, Japanese Laid-open Patent Publication No. 2015-203881). For instance, in the case where waking-up or bed-leaving behavior leading to an accident of falling down or falling off a bed or an abnormal behavior of a patient such as suffering and unable to press a nurse call button occurs in a medical institution, it is effective to notify a nurse of the situation by a system on behalf of the patient. Hereinafter, a patient and a care receiver may be referred to as a subject to be monitored.

There is a demand that the behavior of a subject to be monitored be preferably recognized accurately as the information for determining necessity or a priority order of nursing care, helping care according to a situation of the subject to be monitored as well as a demand that the behavior of the subject to be monitored be preferably shared to achieve continuing care. In order to meet the demands, it is desirable to recognize when a subject to be monitored has exhibited what type of behavior and to present a result of the recognition to healthcare professionals in a plain manner.

A technique is also known, that obtains information indicating only the behavior of a subject to be monitored by identifying mixed behavior in which both the behavior of a subject to be monitored and the behavior of a person other than the subject are present and excluding the behavior of the person from the mixed behavior (see, for instance, Japanese Laid-open Patent Publication No. 2015-210796). In addition, a technique is also known, that detects the head of a subject to be monitored from an image captured using a camera (see, for instance, Japanese Laid-open Patent Publication Nos. 2015-172889, 2015-138460, 2015-186532, and 2015-213537).

In the above-described monitoring system in related art, the situation of a subject to be monitored may be erroneously recognized due to the position of a camera relative to a bed.

This problem arises not only in the case where a patient or a care receiver on a bed is monitored, but also in the case where a healthy human such as a baby is monitored. In consideration of the above, it is desirable that the situation of a subject to be monitored be identified with high accuracy from an image in which a bed appears.

### SUMMARY OF INVENTION

In view of the foregoing, it is an object of the disclosure is to provide a situation identification method, a situation identification device, and a situation identification program that are capable of identifying the situation of a subject to be monitored with high accuracy from an image in which a place to sleep appears.

According to an aspect of the invention, a situation identification method executed by a processor included in a situation identification device, the situation identification method includes acquiring a plurality of images; identifying, for each of the plurality of images, a first area including a bed area where a place to sleep appears in an image, and a second area where an area in a predetermined range around the bed appears in the image; detecting a state of a subject to be monitored for each of the plurality of images based on a result of detection of a head area indicating an area of a head of the subject to be monitored in the first area and a result of detection of a living object in the second area; when the state of the subject to be monitored changes from a first state to a second state, identifying a situation of the subject to be monitored based on a combination of the first state and the second state; and outputting information that indicates the identified situation.

### BRIEF DESCRIPTION OF DRAWINGS

FIG. 1 is a functional configuration diagram of a situation identification device;
FIG. 2 is a flowchart of situation identification processing;
FIG. 3 is a functional configuration diagram illustrating a specific example of the situation identification device;
FIG. 4 is a flowchart illustrating a specific example of situation identification processing;
FIGs. 5A, 5B, and 5C illustrate a first monitoring area;
FIG. 6 illustrates a second monitoring area in a three-dimensional space;
FIG. 7 illustrates the second monitoring area;
FIG. 8 illustrates multiple second monitoring areas set at different positions;
FIG. 9 is a flowchart of bed visitor detection processing;
FIG. 10 is a flowchart of left side detection processing;
FIG. 11 is a flowchart of right side detection processing;
FIGs. 12A, 12B, and 12C illustrate monitoring areas according to installation positions of an imaging device;
FIG. 13 is a flowchart of state detection processing;
FIG. 14 is a flowchart of head area correction processing;
FIG. 15 illustrates a head area at the previous time and the current time;
FIGs. 16A, 16B, and 16C illustrate head area correction processing;
FIG. 17 is a chart illustrating temporal change in state information;
FIG. 18 is a chart illustrating temporal change corresponding to waking-up;
FIG. 19 is a chart illustrating temporal change corresponding to getting up;
FIG. 20 is a chart illustrating temporal change corresponding to leaving a bed alone;
FIG. 21 is a chart illustrating temporal change corresponding to leaving a bed along with a bed visitor;
FIG. 22 is a chart illustrating temporal change corresponding to rolling over;
FIG. 23 is a chart illustrating temporal change corresponding to dangerous behavior;
FIG. 24 is a chart illustrating temporal change corresponding to passing of a bed visitor;
FIG. 25 is a chart illustrating temporal change corresponding to a situation of struggling;
FIG. 26 is a chart illustrating temporal change corresponding to a situation of acting violently;
FIG. 27 a chart illustrating temporal change corresponding to not waking-up;
FIG. 28 is a chart illustrating temporal change corresponding to a static lying posture state for a long time;
FIG. 29 is a chart illustrating temporal change corresponding to falling off;
FIG. 30 is a chart illustrating temporal change corresponding to a situation of being unable to get to sleep;
FIG. 31 illustrates state transitions;
FIG. 32 illustrates a result of detection of the state of a subject to be monitored;
FIG. 33 is a table illustrating the previous state, the current state, and a transition frequency;
FIG. 34A is a flowchart (part one) of state change update processing;
FIG. 34B is a flowchart (part two) of the state change update processing;
FIG. 34C is a flowchart (part three) of the state change update processing;
FIG. 34D is a flowchart (part four) of the state change update processing;
FIG. 35 is a table indicating a situation identification rule for a change from a dynamic lying posture state to a seating posture state;
FIG. 36 is a chart illustrating change from a dynamic lying posture state to a seating posture state;
FIG. 37 is a table indicating the situation identification rule for a change from a seating posture state or a visit state to an absent state;
FIG. 38 is a chart illustrating change from a seating posture state to an absent state;
FIG. 39 is a table indicating the situation identification rule for a change from a seating posture state to an absent state;
FIG. 40 is a table indicating the situation identification rule for the transition frequency;
FIG. 41 is a table indicating the situation identification rule for a change from a static lying posture state to a dynamic lying posture state;
FIG. 42 is a chart illustrating change from a static lying posture state to a dynamic lying posture state;
FIG. 43 is a table indicating the situation identification rule for time;
FIG. 44 is a chart illustrating a dynamic lying posture state for a long time; and
FIG. 45 is a configuration diagram of an information processing device.

### DESCRIPTION OF EMBODIMENTS

Hereinafter, an embodiment will be described in detail with reference to the drawings. Possible situations of a subject to be monitored in a medical institution, a nursing home or the like may include a situation where it is desirable to care for the subject to be monitored, and a situation where the subject to be monitored may not be cared for. The former situation includes behaviors of the subject to be monitored, such as struggling, being unable to get to sleep at night, and acting violently. The latter situation includes behaviors of the subject to be monitored, such as sleeping, being cared for by a healthcare professional.

In the monitoring system described in Japanese Laid-open Patent Publication No. 2015-210796, the area on both sides of a bed is defined as a monitoring area. When motion is detected in the monitoring area, it is determined that a patient is being cared for by a healthcare professional.

However, in a hospital room, it is not necessarily possible to install a camera over the head of a subject to be monitored lying on a bed. The position of the bed may be moved for treatment or care of the subject. For this reason, it is desirable that a camera may be installed at any position relative to the bed.

However, when a camera is installed diagonally relative to the bed or a camera is installed beside the bed, it may be difficult to set a monitoring area due to occlusion by the bed or objects around the bed. When a camera is installed diagonally relative to the bed, the body of a subject to be monitored appears in the monitoring area, and motion of the subject in the monitoring area is detected. It may be erroneously determined by the detection that the subject is being cared for by a healthcare professional. Furthermore, when the area of aisle around the bed is overlapped with the monitoring area, motion of a person passing through the aisle, other than the subject to be monitored is detected. It may also be erroneously determined by the detection that the subject is being cared for by a healthcare professional.

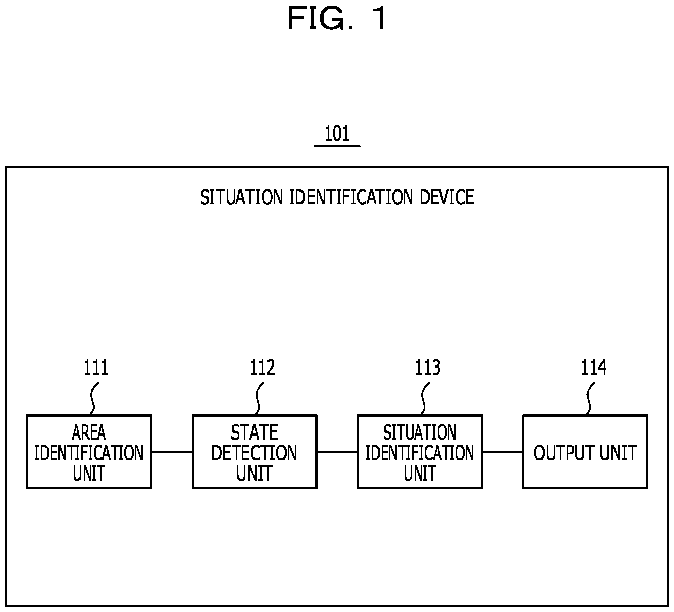

FIG. 1 illustrates a functional configuration example of a situation identification device in an embodiment. A situation identification device 101 of FIG. 1 includes an area identification unit 111, a state detection unit 112, a situation identification unit 113, and an output unit 114.

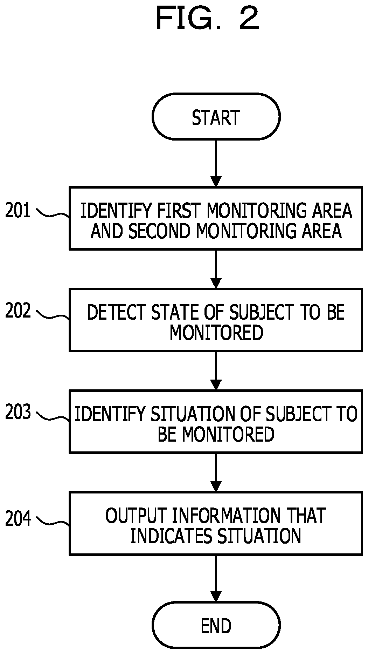

FIG. 2 is a flowchart of an example illustrating situation identification processing performed by the situation identification device 101 of FIG. 1. First, the area identification unit 111 identifies a first monitoring area including a bed area where a bed appears in an image, and a second monitoring area where an area in a predetermined range around the bed appears in the image (operation 201). Next, the state detection unit 112 detects a state of the subject to be monitored for each image based on a result of the detection of a head area in the first monitoring area, and a result of the detection of a living object in the second monitoring area (operation 202).

Next, when the state of the subject to be monitored changes from a first state to a second state, the situation identification unit 113 identifies the situation of the subject to be monitored based on a combination of the first state and the second state (operation 203). The output unit 114 then outputs information that indicates the situation identified by the situation identification unit 113 (operation 204).

The above-described situation identification device 101 enables highly accurate identification of the situation of the subject to be monitored based on the image where the bed appears.

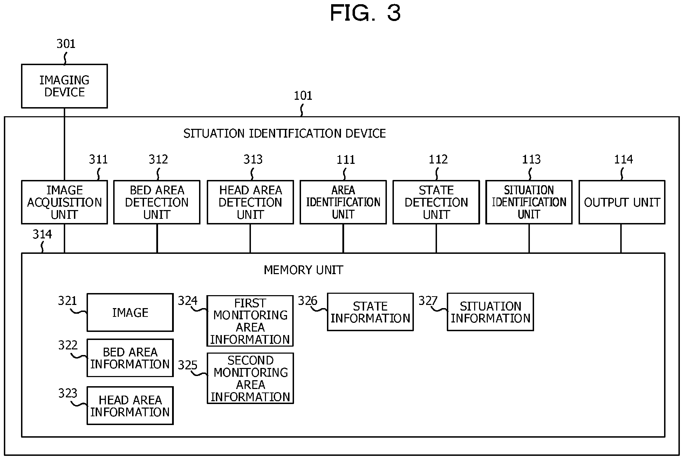

FIG. 3 illustrates a specific example of the situation identification device 101 of FIG. 1. The situation identification device 101 of FIG. 3 includes an area identification unit 111, a state detection unit 112, a situation identification unit 113, an output unit 114, an image acquisition unit 311, a bed area detection unit 312, a head area detection unit 313, and a memory unit 314. The memory unit 314 stores an image 321, bed area information 322, head area information 323, first monitoring area information 324, second monitoring area information 325, state information 326, and situation information 327.

An imaging device 301 is, for instance, a camera and is installed at a location such as the ceiling of a room in which a bed is installed. The imaging device 301 captures an image 321 of a captured area including a bed and a subject to be monitored on the bed, and outputs the image to the situation identification device 101. The image acquisition unit 311 of the situation identification device 101 acquires the image 321 inputted in time series from the imaging device 301, and stores each image in the memory unit 314.

The bed area detection unit 312 detects a bed area where a bed appears from the image 321 at each time. The bed area detection unit 312 then generates bed area information 322 that indicates the detected bed area, and stores the information in the memory unit 314. The head area detection unit 313 detects, from the image 321, a head area where the head of the subject to be monitored appears. The head area detection unit 313 then generates head area information 323 that indicates the detected head area, and stores the information in the memory unit 314.

The area identification unit 111 identifies the first monitoring area in the image 321 using the bed area information 322. The area identification unit 111 then generates first monitoring area information 324 indicating the first monitoring area, and stores the information in the memory unit 314. The area identification unit 111 identifies the second monitoring area in the image 321 using the bed area information 322. The area identification unit 111 then generates second monitoring area information 325 indicating the second monitoring area, and stores the information in the memory unit 314. The first monitoring area is an area for detecting a subject to be monitored on a bed. The second monitoring area is an area for detecting a living object around a bed.

The state detection unit 112 detects a state of a subject to be monitored indicated by the image 321, using the head area information 323, the first monitoring area information 324, and the second monitoring area information 325. The state detection unit 112 then generates state information 326 that indicates the detected state, and stores the information in the memory unit 314. The situation identification unit 113 identifies the situation of the subject to be monitored using the state information 326. The situation identification unit 113 then generates situation information 327 indicating the identified situation, and stores the information in the memory unit 314. The output unit 114 then outputs the situation information 327.

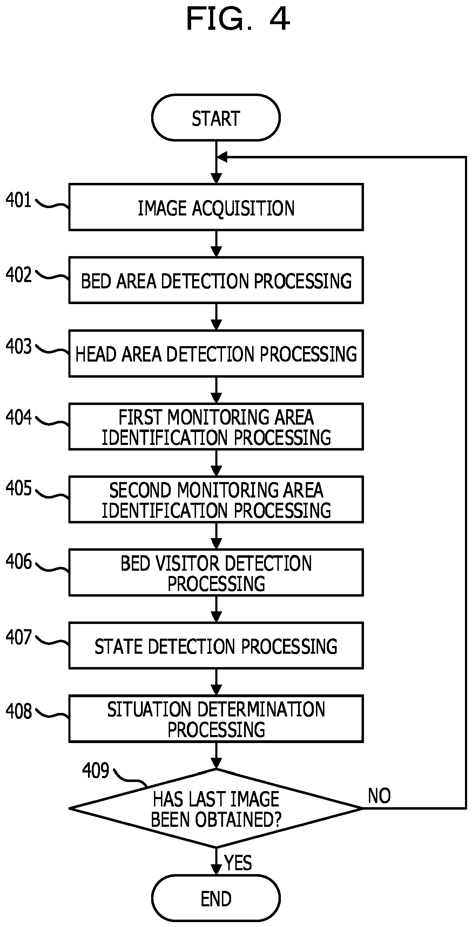

FIG. 4 is a flowchart illustrating a specific example of the situation identification processing performed by the situation identification device 101 of FIG. 3. First, the image acquisition unit 311 acquires the image 321 inputted from the imaging device 301 (operation 401), and the bed area detection unit 312 performs bed area detection processing, and generates the bed area information 322 (operation 402). For instance, the bed area detection unit 312 enables detection of a bed area from the image 321 using the techniques described in Japanese Patent Application Nos. 2014-250795, 2015-219080, and 2016-082864 in previous applications.

In the technique of Japanese Patent Application No. 2014-250795, line segments, by which a bed area is formable, are extracted from the line segments specified by the edges detected from the image 321. Then L-character shapes are each generated by combining two line segments. Subsequently, L-character shapes, by which the bed area is formable, are extracted, U-character shapes are each generated by combining two L-character shapes, and U-character shapes, by which the bed area is formable, are extracted. A rectangular shape is then generated by combining two U-character shapes, and the rectangular shape that represents the bed area is extracted.

In the technique of Japanese Patent Application No. 2015-219080, the line segments detected from the image 321 are converted into line segments in a three-dimensional space, then a rectangular shape representing a bed area is extracted by a method similar to the method of Japanese Patent Application No. 2014-250795.

In the technique of Japanese Patent Application No. 2016-082864, part of a bed candidate area in the image 321 is identified based on the line segments extracted from the image 321, and a search range for a line segment is set based on a new bed candidate area which is set based on the part of the bed candidate area. The set bed candidate area is corrected based on the placement of the line segments included in the search range.

Next, the head area detection unit 313 performs head area detection processing, and generates the head area information 323 (operation 403). For instance, the head area detection unit 313 enables detection of a head area from the image 321 using the techniques described in Japanese Laid-open Patent Publication No. 2015-172889, Japanese Laid-open Patent Publication No. 2015-138460, Japanese Laid-open Patent Publication No. 2015-186532, Japanese Laid-open Patent Publication No. 2015-213537, and Japanese Patent Application No. 2015-195275.

In the technique of Japanese Patent Application No. 2015-195275, a candidate for the head of a subject to be monitored 603 is searched in an area to be monitored in the image 321. When a candidate for the head is not detected in a second area but is detected in a first area out of the first area and the second area in the area to be monitored, a candidate at the uppermost position in the first area is detected as the head.

Next, the area identification unit 111 performs first monitoring area identification processing using the bed area information 322, and thereby generates the first monitoring area information 324 (operation 404), then performs second monitoring area identification processing, and thereby generates the second monitoring area information 325 (operation 405).

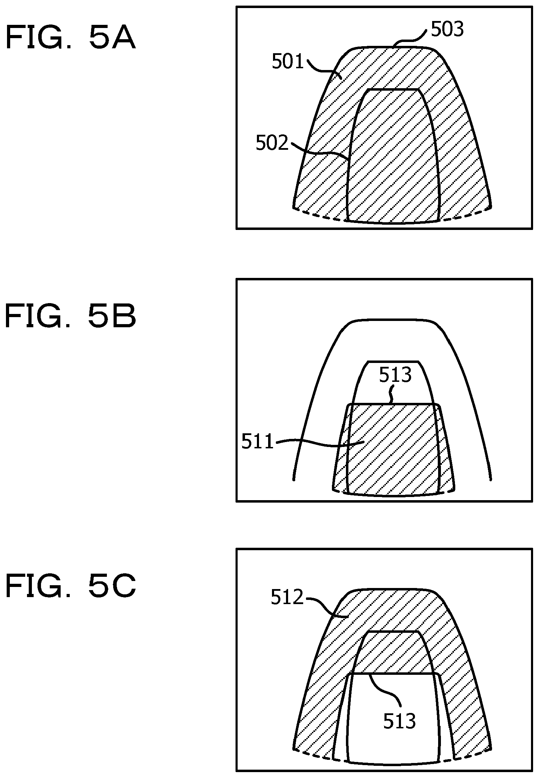

FIGs. 5A, 5B, and 5C illustrate an example of the first monitoring area identified by the first monitoring area identification processing of the operation 404. An area 501 of FIG. 5A indicates the first monitoring area, and includes a bed area 502. A boundary line 503 between the area 501 and the background area indicates a bed-leaving determination line.

For instance, the bed-leaving determination line is set to a position over the top of the head of the subject to be monitored in a three-dimensional space in a state where the subject to be monitored is sitting up on a bed, and the bed-leaving determination line is mapped onto the image 321. When the head of the subject to be monitored has moved to the outside of the area 501 across the boundary line 503 in the image 321, it is determined that the subject to be monitored has left the bed.

An area 511 of FIG. 5B indicates a lying posture area included in the first monitoring area, and an area 512 of FIG. 5C indicates a seating posture area included in the first monitoring area. A boundary line 513 between the area 511 and the area 512 indicates a wake-up determination line.

For instance, the wake-up determination line is set to a position under the head of the subject to be monitored in a three-dimensional space in a state where the subject to be monitored is sitting up on a bed, and the wake-up determination line is mapped onto the image 321. When the head of the subject to be monitored has moved from the area 511 to the area 512 across the boundary line 513 in the image 321, it is determined that the subject to be monitored has woken up.

Thus, when the head is present in the area 511, it is estimated that the subject to be monitored lies on the bed, and when the head is present in the area 512, it is estimated that the subject to be monitored is sitting on the bed. The bed-leaving determination line and the wake-up determination line may be set using the technique described in Japanese Patent Application No. 2015-195275, for instance.

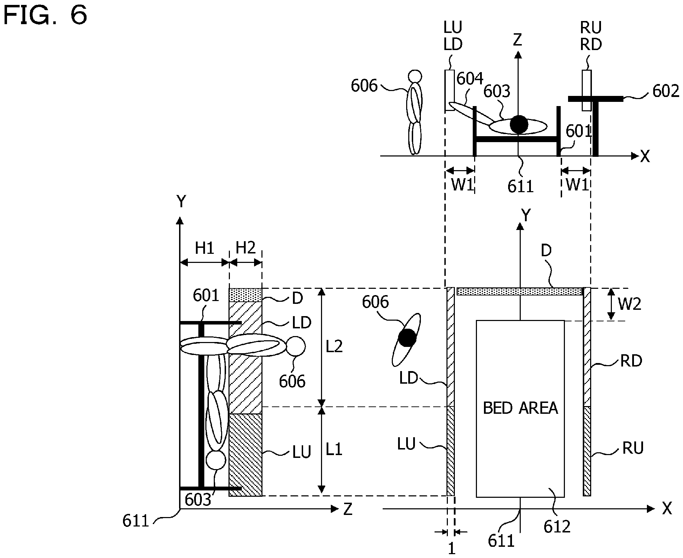

FIG. 6 illustrates an example of the second monitoring area set in a three-dimensional space by the second monitoring area identification processing of the operation 405. The three-dimensional space in a room where the bed is installed is represented by an XYZ coordinate system with the origin at an intersection point 611 between the floor and a perpendicular line from the installation position of the imaging device 301 to the floor. The X-axis is parallel to the short side of a bed area 612 corresponding to a bed 601. The Y-axis is parallel to the long side of the bed area 612. The Z-axis includes the perpendicular line from the installation position of the imaging device 301 to the floor. Thus, the imaging device 301 is installed on the Z-axis.

A subject to be monitored 603 lies on the bed 601. A table 602 is installed beside the bed 601. A bed visitor 606 may enter the room and the subject to be monitored 603 may stretch out an arm 604 to the bed visitor 606. The bed visitor 606 indicates a bed visitor to the bed 601. The bed visitor 606 is, for instance, a nurse who nurses the subject to-be-monitored, a care worker who cares for the subject to-be-monitored, a doctor who treats the subject to-be-monitored, a visitor, and another subject to-be-monitored who shares the same room with the subject to-be-monitored.

In this example, the second monitoring area in the three-dimensional space includes five areas: an area LU, an area LD, an area D, an area RD, and an area RU. The second monitoring area is used to detect the bed visitor 606. Each of the areas is a rectangular shape having a thickness of 1 (unit length). The area LU and the area LD are set on the left side of the subject to be monitored 603. The area RU and the area RD are set on the right side of the subject to be monitored 603. The area D is set on the leg side of the subject to be monitored 603. The area LU is set closer to the head of the subject to be monitored 603 than the area LD is. The area RU is set closer to the head of the subject to be monitored 603 than the area RD is.

Each area is set at a position away from the XY-plane by H1 in the Z-axis direction. Each area has a height H2 in the Z-axis direction. The area LU and the area RU each have a width L1 in the Y-axis direction. The area LD and the area RD each have a width L2 in the Y-axis direction. The area LU and the area LD are set at a position away from the long side of the bed area 612 on the left side by W1 in the X-axis direction. The area RU and the area RD are set at a position away from the long side of the bed area 612 on the right side by W1 in the X-axis direction. The area D is set at a position away from the short side of the bed area 612 on the leg side by W2 in the Y-axis direction.

The values of H1, H2, L1, L2, W1, and W2 may be designated by a user. The area identification unit 111 may set those values by a predetermined algorithm.

An effect of occlusion due to the bed 601 and the table 602 is avoidable by setting the second monitoring area on both sides of the bed 601 at predetermined height positions. Setting the upper end of the second monitoring area over the bed-leaving determination line reduces the possibility of detecting motion of the subject to be monitored 603 in the second monitoring area. Thus, the bed visitor 606 approaching the bed 601 in a standing posture and the bed visitor 606 leaving the bed 601 in a standing posture are detectable with high accuracy.

Furthermore, dividing the second monitoring area into multiple areas makes it possible to distinguish whether the bed visitor 606 is approaching the bed 601, leaving the bed 601, or the subject to be monitored 603 is being cared for, based on the presence or absence of a living object in each area.

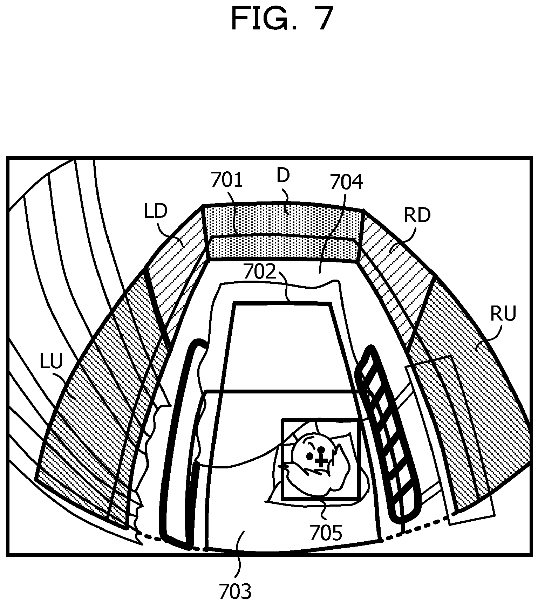

FIG. 7 illustrates an example of the second monitoring area obtained by mapping the second monitoring area of FIG. 6 from the XYZ coordinate system to an image coordinate system on the image 321. An area 701 corresponds to the first monitoring area, and includes a bed area 702. An area 703 and an area 704 correspond to a lying posture area and a seating posture area, respectively. An area LU, an area LD, an area D, an area RD, and an area RU correspond to the second monitoring area. An area 705 corresponds to the head area.

Next, the state detection unit 112 performs bed visitor detection processing using the second monitoring area information 325 (operation 406). The state detection unit 112 generates state information 326 by performing state detection processing using the head area information 323 and the first monitoring area information 324 (operation 407).

Subsequently, the situation identification unit 113 generates situation information 327 by performing situation determination processing using the state information 326. The output unit 114 then outputs the situation information 327 (operation 408). The image acquisition unit 311 checks whether or not the last image 321 inputted from the imaging device 301 has been obtained (operation 409).

When the last image 321 has not been obtained (NO in the operation 409), situation identification device 101 repeats the processing on and after the operation 401. When the last image 321 has been obtained (YES in the operation 409), the situation identification device 101 completes the processing.

With this situation identification processing, even in hours such as at night when the subject to be monitored 603 such as a patient or a care receiver stays in a room alone and the state of the subject is difficult to be recognized, it is possible to identify the situation of the subject to be monitored 603 with high accuracy and to notify a healthcare professional of the situation. Therefore, the healthcare professional is able to recognize the situation of the subject to be monitored 603 accurately.

The situation identification device 101 may perform the processing of the operations 401 to 408 every predetermined time instead of for each image (each frame). In this case, for instance, several hundreds ms to several seconds may be used as the predetermined time.

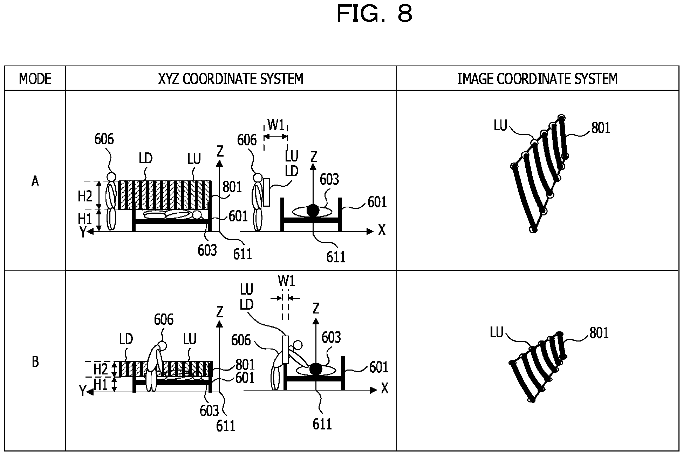

FIG. 8 illustrates multiple second monitoring areas set at different positions. In mode A, W1 is set to several tens cm to approximately 1 m in order to detect the bed visitor 606 approaching the bed 601 in a standing posture and the bed visitor 606 leaving the bed 601 in a standing posture around the bed 601. H1 is set to approximately the length of a human leg. H2 is set to approximately the length of the human trunk.

On the other hand, in mode B, W1, H1, and H2 are set to smaller values than those in mode A in order to detect the bed visitor 606 who is caring for the subject to be monitored 603 in a standing posture or in a semi-crouching posture around the bed 601.

Multiple vertically long detection areas 801 are provided in the area LD and the area LU. The state detection unit 112 performs bed visitor detection processing using the pixel values of pixels in the detection areas 801 in the image coordinate system. Similar detection areas 801 are also provided in the area D, the area RD, and the area RU.

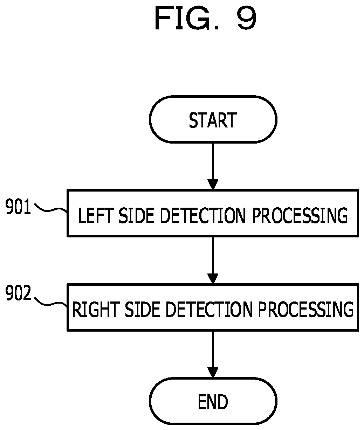

FIG. 9 is a flowchart illustrating an example of bed visitor detection processing in the operation 406 of FIG. 4. First, the state detection unit 112 performs left side detection processing to detect the bed visitor 606 on the left side of the subject to be monitored 603 (operation 901). The state detection unit 112 then records left-side bed visitor information on the memory unit 314, the left-side bed visitor information indicating the presence or absence of the bed visitor 606 on the left side.

Subsequently, the state detection unit 112 performs right side detection processing to detect the bed visitor 606 on the right side of the subject to be monitored 603 (operation 902). The state detection unit 112 then records right-side bed visitor information on the memory unit 314, the right-side bed visitor information indicating the presence or absence of the bed visitor 606 on the right side.

At the start of the situation identification processing, in the left-side bed visitor information and the right-side bed visitor information, absence of a bed visitor is set. The mode of the second monitoring area is set to mode A.

At the start of the situation identification processing, in the left-side bed visitor information and the right-side bed visitor information, absence of a bed visitor is set. The mode of the second monitoring area is set to mode A.

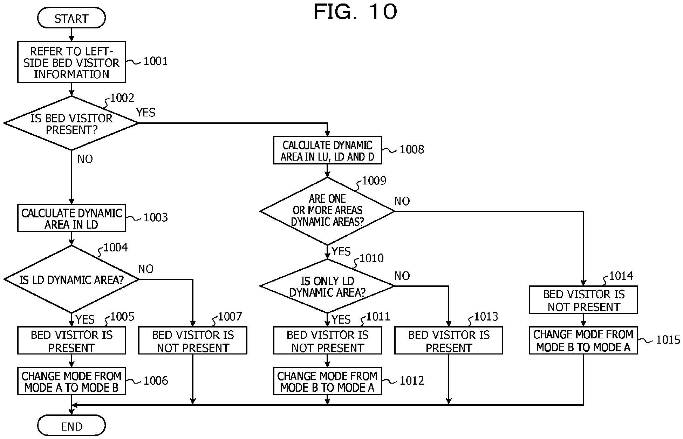

FIG. 10 is a flowchart illustrating an example of the left side detection processing in the operation 901 of FIG. 9. In the left side detection processing, a dynamic area in the image 321 is calculated using, for instance, the technique described in Japanese Laid-open Patent Publication No. 2015-203881 or 2015-210796. When a dynamic area is present in a certain area, it is determined that a living object appears in the certain area. When a dynamic area is not present in a certain area, it is determined that a living object does not appear in the certain area.

First, the state detection unit 112 refers to the left-side bed visitor information recorded for the image 321 at the previous time (operation 1001), and checks the presence or absence of the bed visitor 606 at the previous time (operation 1002).

When the bed visitor 606 is not present at the previous time (NO in the operation 1002), the state detection unit 112 calculates a dynamic area in a detection area 801 provided in the area LD (operation 1003), and checks whether or not a dynamic area is present in the area LD (operation 1004).

When a dynamic area is present in the area LD (YES in the operation 1004), the state detection unit 112 determines that the bed visitor 606 is present at the current time, and records the presence of the bed visitor 606 on the left-side bed visitor information (operation 1005). The state detection unit 112 then changes the mode of the second monitoring area from mode A to mode B (operation 1006). Thus, it is possible to detect that the bed visitor 606 who has entered the room is approaching the bed 601 from the left leg side of the subject to be monitored 603.

On the other hand, when a dynamic area is not present in the area LD (NO in the operation 1004), the state detection unit 112 determines that the bed visitor 606 is not present at the current time. The state detection unit 112 records the absence of the bed visitor 606 on the left-side bed visitor information (operation 1007).

When the bed visitor 606 is present at the previous time (YES in the operation 1002), the state detection unit 112 calculates a dynamic area in the detection area 801 provided in the area LU, the area LD, and the area D (operation 1008). The state detection unit 112 then checks whether or not a dynamic area is present in at least one or more of the area LU, the area LD, and the area D (operation 1009). When a dynamic area is present in one or more areas (YES in the operation 1009), the state detection unit 112 checks whether or not a dynamic area is present only in the area LD (operation 1010).

When a dynamic area is present only in the area LD (YES in the operation 1010), the state detection unit 112 determines that the bed visitor 606 is not present, and records the absence of the bed visitor 606 on the left-side bed visitor information (operation 1011). The state detection unit 112 then changes the mode of the second monitoring area from mode B to mode A (operation 1012). Thus, it is possible to detect that the bed visitor 606 who has finished caring for the subject to be monitored 603 leaves the bed 601 from the left leg side of the subject to be monitored 603.

On the other hand, when a dynamic area is present in an area other than the area LD (NO in the operation 1010), the state detection unit 112 determines that the bed visitor 606 is present. The state detection unit 112 records the presence of the bed visitor 606 on the left-side bed visitor information (operation 1013). Thus, it is possible to detect that the bed visitor 606 is continuing to care for the subject to be monitored 603.

When a dynamic area is present in none of the area LU, the area LD, and the area D (NO in the operation 1009), the state detection unit 112 determines that the bed visitor 606 is not present. The state detection unit 112 then records the absence of the bed visitor 606 on the left-side bed visitor information (operation 1014). The state detection unit 112 then changes the mode of the second monitoring area from mode B to mode A (operation 1015). Thus, it is possible to detect that the bed visitor 606 who has finished caring for the subject to be monitored 603 has already left the bed 601.

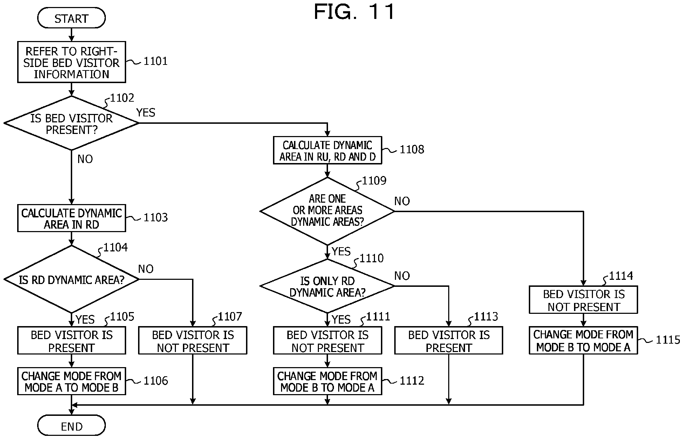

FIG. 11 is a flowchart illustrating an example of right side detection processing in the operation 902 of FIG. 9. Right side detection processing of FIG. 11 is processing such that the left-side bed visitor information is replaced by right-side bed visitor information, and the area LD and the area LU are replaced by the area RD and the area RU, respectively in the left side detection processing of FIG. 10. Performing the right side detection processing making it possible to detect that the bed visitor 606 is approaching the bed 601 from the right leg side of the subject to be monitored 603, and the bed visitor 606 is leaving the bed 601 from the right leg side of the subject to be monitored 603.

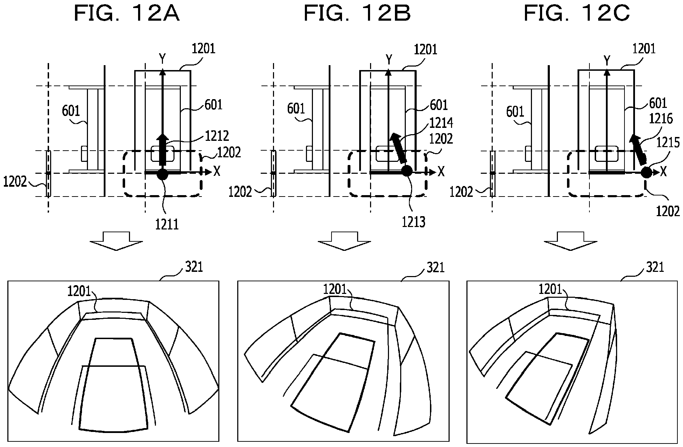

FIGs. 12A, 12B, and 12C illustrate an example of a monitoring area according to the installation position of the imaging device 301. A first monitoring area 1201 corresponds to the area 501 of FIG. 5. A possible installation range 1202 indicates a range in which the imaging device 301 is allowed to be installed. When a wide angle camera is used as the imaging device 301, distortion occurs in the image 321, and the position and shape the first monitoring area 1201 in the image 321 vary with the position and orientation of the imaging device 301.

In the example of FIG. 12A, the imaging device 301 is installed in the orientation indicated by an arrow 1212 at a position 1211 on the intersection line between a plane corresponding to the possible installation range 1202, and the YZ-plane. In this case, the first monitoring area 1201 and the second monitoring area are symmetrical in the image 321.

In the example of FIG. 12B, the imaging device 301 is installed in the orientation indicated by an arrow 1214 at a position 1213 a short distance away from the intersection line in the X-axis direction. In this case, the first monitoring area 1201 and the second monitoring area are somewhat more distorted in the image 321 than in FIG. 12A.

In the example of FIG. 12C, the imaging device 301 is installed in the orientation indicated by an arrow 1216 at a position 1215 on the outer periphery of the possible installation range 1202. In this case, the first monitoring area 1201 and the second monitoring area are much more distorted in the image 321 than in FIG. 12B.

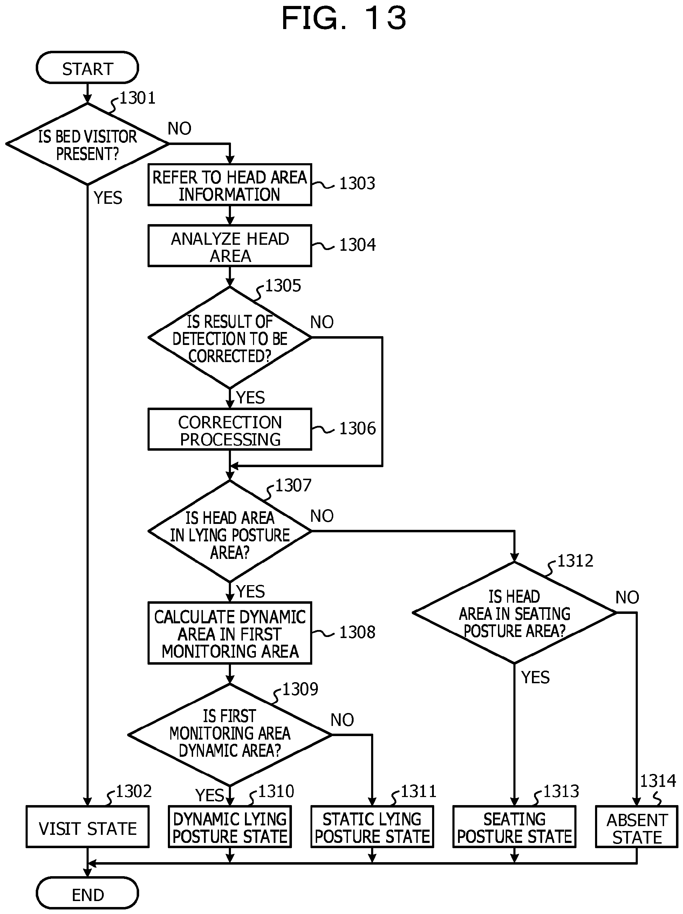

FIG. 13 is a flowchart illustrating an example of state detection processing in the operation 407 of FIG. 4. In the state detection processing, the state of the subject to be monitored 603 indicated by the image 321 is determined to be corresponding to one of the following states:
(1) a visit state: a state where the bed visitor 606 is present. (2) a dynamic lying posture state: a state where the subject to be monitored 603 is lying and moving on the bed 601. (3) a static lying posture state: a state where the subject to be monitored 603 is lying and not moving on the bed 601. (4) a seating posture state: a state where the subject to be monitored 603 is sitting on the bed 601. (5) an absent state: a state where the subject to be monitored 603 is not present on the bed 601.

It is highly probable that the body other than the head of the subject to be monitored 603 on the bed 601 is covered by bedding. Thus, the state detection unit 112 determines whether or not the subject to be monitored 603 is present on the bed 601 from the relative positional relationship between the bed 601 and the head of the subject to be monitored 603. When the subject to be monitored 603 is present on the bed 601, the state detection unit 112 then determines whether the subject to be monitored 603 is lying or sitting from the relative positional relationship between the bed 601 and the head. In addition, the state detection unit 112 classifies a lying posture state into a dynamic lying posture state and a static lying posture state based on the presence or absence of a dynamic area in the lying posture area.

First, the state detection unit 112 refers to the right-side bed visitor information and the left-side bed visitor information recorded in the operation 406 to check whether the bed visitor 606 is present on the right side or the left side (operation 1301). When the bed visitor 606 is present (YES in the operation 1301), the state detection unit 112 determines that the state of the subject to be monitored 603 is a visit state, and generates state information 326 that indicates a visit state (operation 1302).

On the other hand, when the bed visitor 606 is not present (NO in the operation 1301), the state detection unit 112 refers to the head area information 323 (operation 1303), and analyzes the head area indicated by the head area information 323 (operation 1304). The state detection unit 112 then determines whether or not a result of detection of the head area is to be corrected (operation 1305).

When the result of detection of the head area is corrected (YES in the operation 1305), the state detection unit 112 performs correction processing (operation 1306). On the other hand, when the result of detection of the head area is not corrected (NO in the operation 1305), the state detection unit 112 skips the correction processing.

Subsequently, the state detection unit 112 checks whether or not the head area is present in the lying posture area included in the first monitoring area (operation 1307). When the head area is present in the lying posture area (YES in the operation 1307), the state detection unit 112 calculates a dynamic area in the first monitoring area (operation 1308). The state detection unit 112 then checks whether or not a dynamic area is present in the first monitoring area (operation 1309).

When a dynamic area is present in the first monitoring area (YES in the operation 1309), the state detection unit 112 determines that the state of the subject to be monitored 603 is a dynamic lying posture state. The state detection unit 112 then generates state information 326 that indicates a dynamic lying posture state (operation 1310). On the other hand, when a dynamic area is not present in the first monitoring area (NO in the operation 1309), the state detection unit 112 determines that the state of the subject to be monitored 603 is a static lying posture state. The state detection unit 112 then generates state information 326 that indicates a static lying posture state (operation 1311).

When the head area is not present in the lying posture area (NO in the operation 1307), the state detection unit 112 checks whether or not the head area is present in the seating posture area included in the first monitoring area (operation 1312). When the head area is present in the seating posture area (YES in the operation 1312), the state detection unit 112 determines that the state of the subject to be monitored 603 is a seating posture state. The state detection unit 112 then generates state information 326 that indicates a seating posture state (operation 1313).

On the other hand, when the head area is not present in the seating posture area (NO in the operation 1312), the state detection unit 112 determines that the state of the subject to be monitored 603 is an absent state. The state detection unit 112 then generates state information 326 that indicates an absent state (operation 1314).

A dynamic lying posture state, a static lying posture state, and a seating posture state correspond to a present state where the subject to be monitored 603 is present on the bed 601. When the head area is present in a lying posture area or a seating posture area, the head area is present in the first monitoring area. Therefore, the state detection unit 112 performs the processing of the operation 1307 and the operation 1312, and thereby detects a present state when the head area is present in the first monitoring area, or detects an absent state when the head area is not present in the first monitoring area.

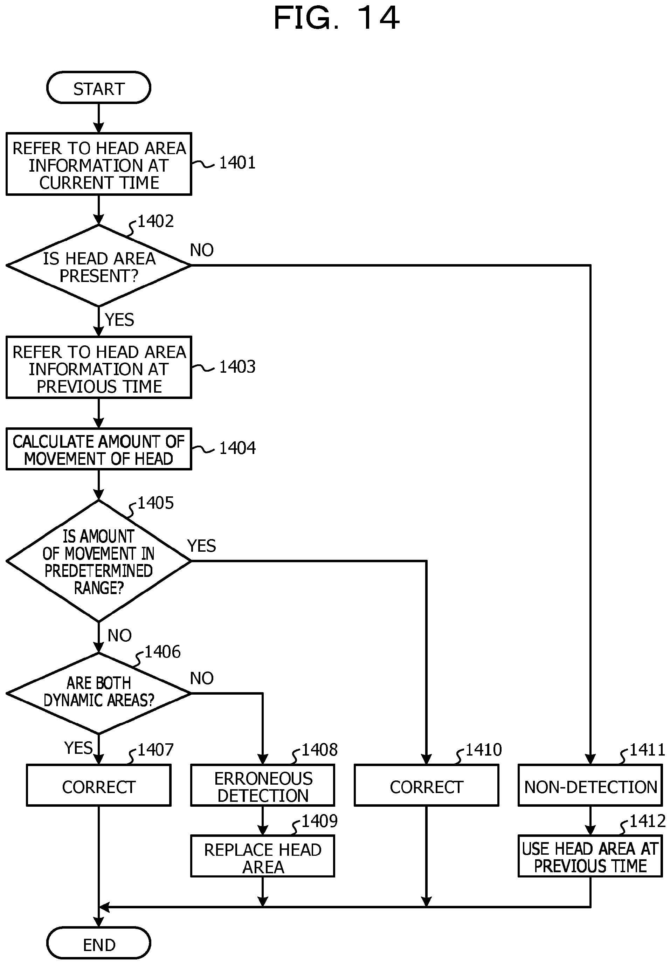

FIG. 14 is a flowchart illustrating an example of head area correction processing corresponding to the operation 1303 to the operation 1306 of FIG. 13.

In a head recognition technique using histogram of oriented gradients (HOG), an area having a characteristic quantity most similar to learned data of the head is detected as the head area. For this reason, when an area having a characteristic quantity more similar to the learned data is present, such as wrinkles in clothes, wrinkles in a sheet, wrinkles or patterns in bedding, a shoulder portion, the area may be erroneously detected as the head area. When the head appears with a low brightness gradient, the head area may not be detected. Thus, the state detection unit 112 corrects erroneous detection or non-detection of the head area by performing the head area correction processing.

First, the state detection unit 112 refers to the head area information 323 at the current time (operation 1401). The state detection unit 112 then checks whether or not the head area is present in the image 321 at the current time (operation 1402).

When the head area is present in the image 321 at the current time (YES in the operation 1402), the state detection unit 112 refers to the head area information 323 at the previous time (operation 1403). The state detection unit 112 then calculates an amount of movement of the head between the previous time and the current time (operation 1404). The state detection unit 112 then checks whether or not the amount of movement of the head is in a predetermined range (operation 1405). For instance, the amount of movement of the head is expressed by the relative position of the head area in the image 321 at the current time with respect to the head area in the image 321 at the previous time. The predetermined range is determined based on the head area at the previous time.

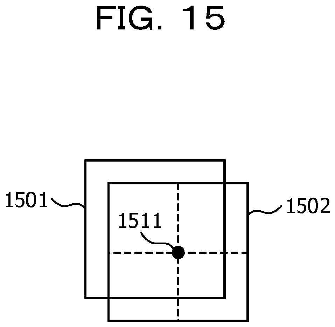

FIG. 15 illustrates an example of the head areas at the previous time and the current time. In this case, a head area 1501 at the previous time (t-1) is used as the predetermined range. When a center 1511 of a head area 1502 at the current time t is present in the head area 1501, the amount of movement is determined to be within the predetermined range. On the other hand, when the center 1511 is not present in the head area 1501, the amount of movement is determined to be out of the predetermined range.

When the amount of movement is out of the predetermined range (NO in the operation 1405), the state detection unit 112 checks whether or not the head area at the previous time and the head area at the current time are both dynamic areas (operation 1406). When both head areas are dynamic areas (YES in the operation 1406), the state detection unit 112 determines that the result of detection of the head area at the current time is correct (operation 1407), and does not correct the result of detection.

On the other hand, when at least one of the head area at the previous time and the head area at the current time is not a dynamic area (NO in the operation 1406), the state detection unit 112 determines that the head area at the current time has been erroneously detected (operation 1408). The state detection unit 112 then generates head area information 323 in which the head area at the current time is replaced by the head area at the previous time (operation 1409).

When the amount of movement is within the predetermined range (YES in the operation 1405), the state detection unit 112 determines that the result of detection of the head area at the current time is correct (operation 1410), and does not correct the result of detection.

When the head area is not present in the image 321 at the present time (NO in the operation 1402), the state detection unit 112 determines that the head area at the current time has not been detected (operation 1411). The state detection unit 112 then generates head area information 323 in which the head area at the previous time is used as the head area at the current time (operation 1412).

In the operation 1411, when the head area at the previous time is not a dynamic area but a static area, the state detection unit 112 may determine that the head area at the current time has not been detected. This is because when the head area at the previous time is a static area, the head has not moved at the previous time, and thus it is highly probable that the head is present at the same position also at the current time.

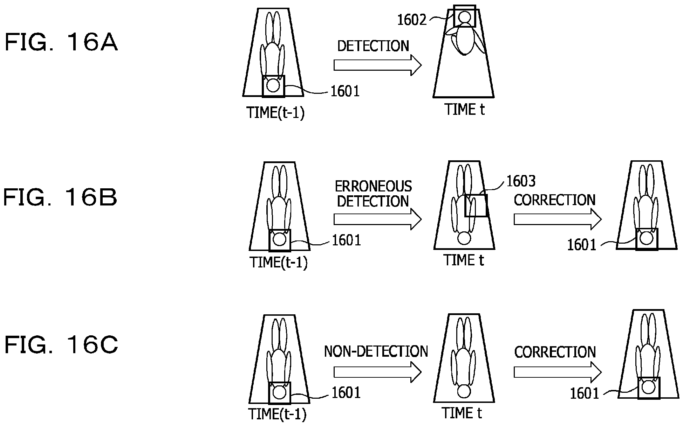

FIGs. 16A, 16B, and, 16C illustrate an example of the head area correction processing. FIG. 16A illustrates the case where the result of detection is determined to be correct in the operation 1407. When the head area 1601 at the previous time (t - 1) and the head area 1602 at the current time t are both dynamic areas, and the amount of movement is out of the predetermined range, it may be estimated that the head area 1601 has moved due to a movement of the head between time (t - 1) and time t. Therefore, the result of detection of the head area 1602 is determined to be correct.

FIG. 16B illustrates the case where it is determined that the head area has been erroneously detected in the operation 1408. When the head area 1601 at the previous time (t - 1) is a static area, a head area 1603 at time t is a dynamic area, and the amount of movement is out of the predetermined range, it may be estimated that the head area 1601 has moved by recognizing another object similar to the head as the head at time t. Therefore, the result of detection of the head area 1603 is determined to be incorrect. Thus, the head area 1603 is replaced by the head area 1601.

FIG. 16C illustrates the case where the head area has not been detected in the operation 1411. When the head area 1601 at the previous time (t - 1) is a static area, the head area is not detected at time t, it may be estimated that the head has not moved and is present at the same position at time (t - 1). Therefore, it is determined that the head area has not been detected. The head area 1601 is used as the head area at time t.

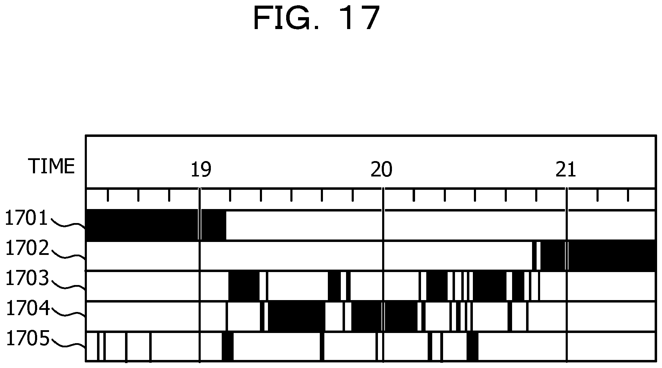

FIG. 17 illustrates an example of temporal change in the state information 326 generated by the state detection processing of FIG. 13. A state change 1701 indicates temporal change in the absent state. A state change 1702 indicates temporal change in the static lying posture state. A state change 1703 indicates temporal change in the dynamic lying posture state. A state change 1704 indicates temporal change in the seating posture state. A state change 1705 indicates temporal change in the visit state.

The output unit 114 may visualize the state information 326 by displaying a temporal change as illustrated in FIG. 17 on a screen and may present the state information 326 to a healthcare professional. In the operation 408 of FIG. 4, the situation identification unit 113 may determine a generation time, a duration time, and a transition frequency in each state from the temporal change in the state information 326, and may identify the situation of the subject to be monitored 603 based on the determined information.

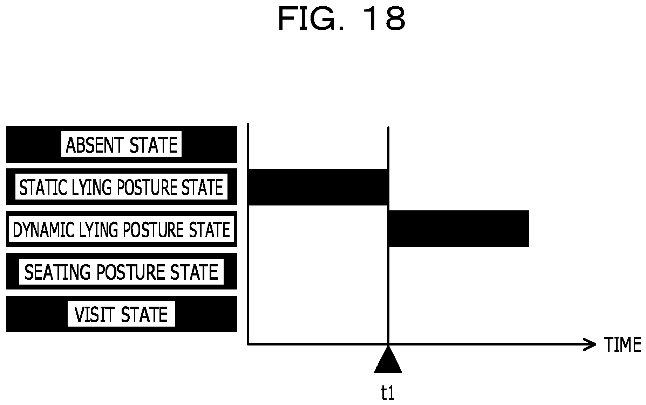

FIGs. 18 to 30 illustrate an example of temporal change in the state information 326 corresponding to specific situations of the subject to be monitored 603. FIG. 18 illustrates an example of temporal change corresponding to waking-up. When the state of the subject to be monitored 603 changes from a static lying posture state to a dynamic lying posture state at time t1, the situation identification unit 113 determines that the subject to be monitored 603 is in wake-up situation.

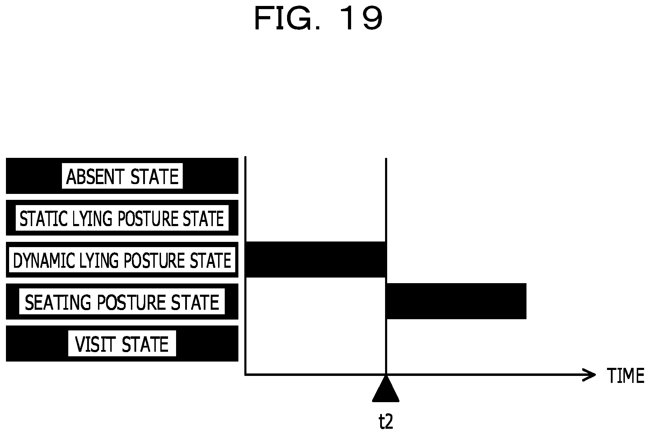

FIG. 19 illustrates an example of temporal change corresponding to getting up. When the state of the subject to be monitored 603 changes from a dynamic lying posture state to a seating posture state at time t2, the situation identification unit 113 determines that the subject to be monitored 603 is in getting up situation on the bed 601.

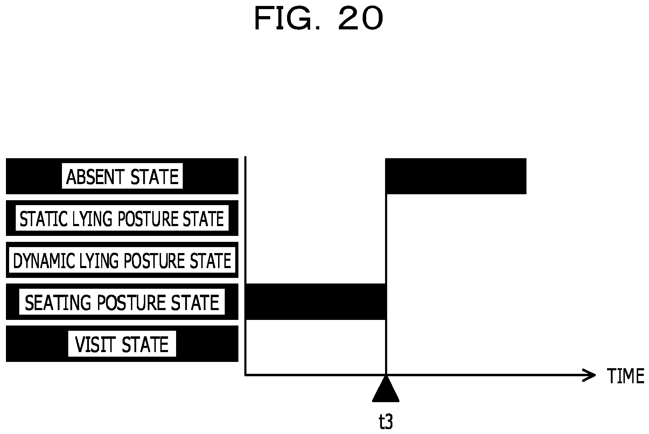

FIG. 20 illustrates an example of temporal change corresponding to leaving a bed alone. When the state of the subject to be monitored 603 changes from a seating posture state to an absent state at time t3, the situation identification unit 113 determines that the subject to be monitored 603 is in a situation where the subject has left the bed 601 alone. In this case, when an absent state continues a predetermined time or longer, the situation identification unit 113 notifies a healthcare professional of the leaving a bed alone.

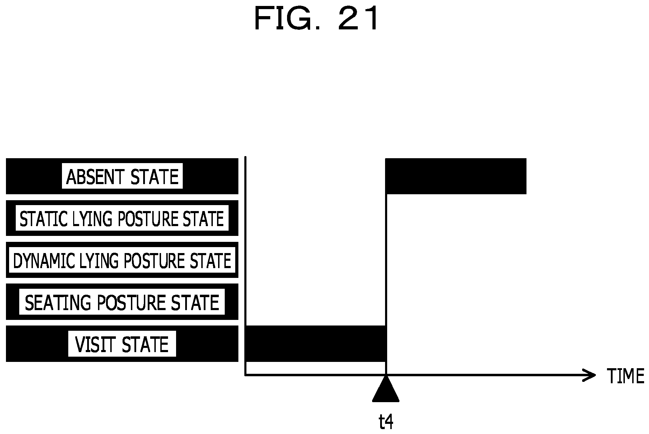

FIG. 21 illustrates an example of temporal change corresponding to leaving the bed along with the bed visitor 606. When the state of the subject to be monitored 603 changes from a visit state to an absent state at time t4, the situation identification unit 113 determines that the subject to be monitored 603 is in a situation where the subject has left the bed 601 along with the bed visitor 606. In this case, the situation identification unit 113 does not notify a healthcare professional of the leaving the bed even when an absent state continues a predetermined time or longer.

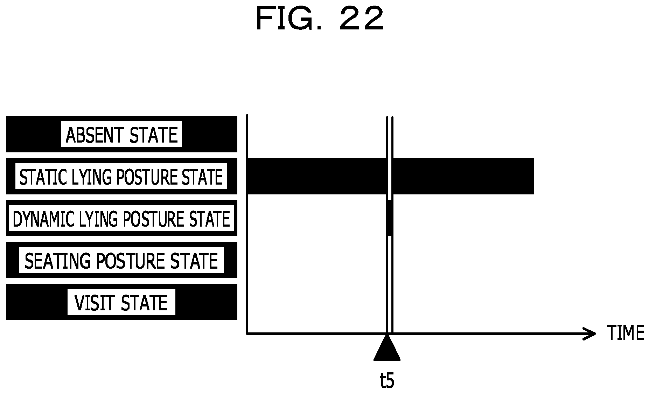

FIG. 22 illustrates an example of temporal change corresponding to rolling over. When the state of the subject to be monitored 603 changes from a static lying posture state to a dynamic lying posture state at time t5, then immediately changes back to a static lying posture state, the situation identification unit 113 determines that the subject to be monitored 603 is in a situation where the subject has rolled over on the bed 601.

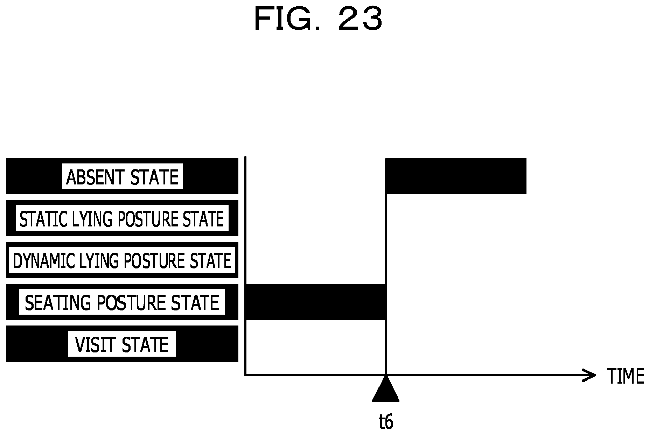

FIG. 23 illustrates an example of temporal change corresponding to dangerous behavior. When the state of the subject to be monitored 603 changes from a seating posture state to an absent state at time t6, the situation identification unit 113 determines that the subject to be monitored 603 is in a situation where the subject has stood up on the bed 601. In this case, the situation identification unit 113 notifies a healthcare professional of the dangerous behavior.

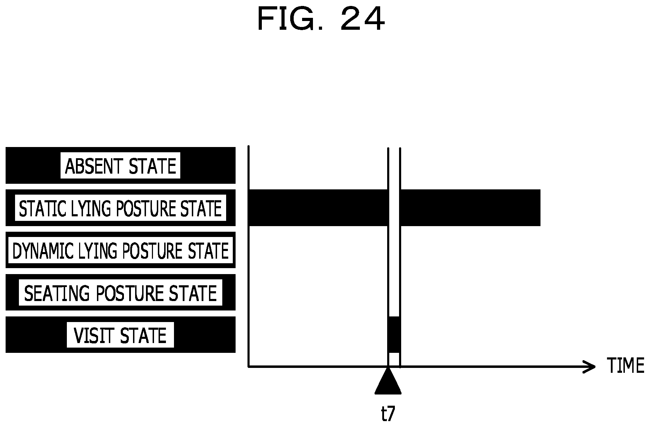

FIG. 24 illustrates an example of temporal change corresponding to passing of the bed visitor 606. When the state of the subject to be monitored 603 changes from a static lying posture state to a visit state at time t7, then changes back to a static lying posture state in a short time, the situation identification unit 113 determines that the subject to be monitored 603 is in a situation where the bed visitor 606 has passed while the subject is sleeping. In this case, it is estimated that the bed visitor 606 is a healthcare professional who makes regular look-around or regular care.

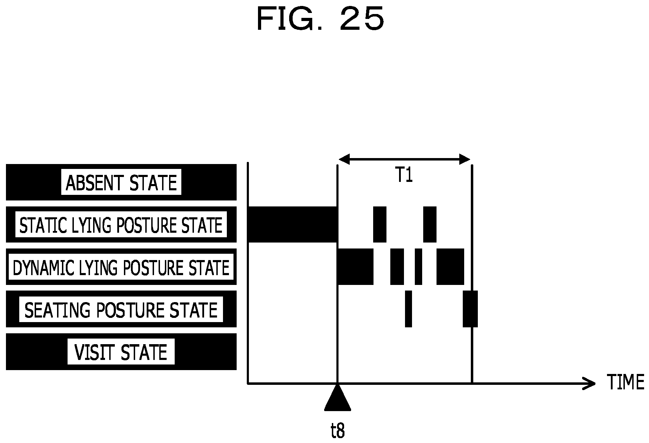

FIG. 25 illustrates an example of temporal change corresponding to a situation of struggling. When the state of the subject to be monitored 603 changes from a static lying posture state to a dynamic lying posture state at time t8, then state change to a static lying posture state, a dynamic lying posture state, and a seating posture state are repeated during a period T1. Thus, when the duration time in each state is less than or equal to a predetermined time during the period T1, and the number of state changes is greater than a predetermined number, the situation identification unit 113 determines that the subject to be monitored 603 is in a situation of struggling. In this case, the situation identification unit 113 notifies a healthcare professional of an abnormal behavior.

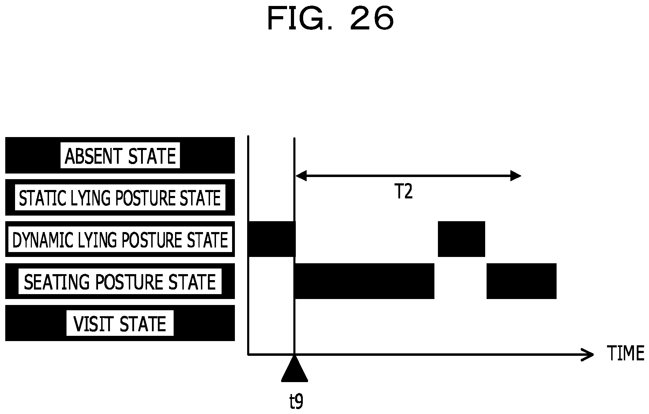

FIG. 26 illustrates an example of temporal change corresponding to a situation of acting violently. When the state of the subject to be monitored 603 changes from a dynamic lying posture state to a seating posture state at time t9, then state change to a dynamic lying posture state and a seating posture state are repeated during a period T2. Thus, when the duration time in each state is less than or equal to a predetermined range during the period T2, and the number of state changes is greater than a predetermined number, the situation identification unit 113 determines that the subject to be monitored 603 is in a situation of acting violently. In this case, the situation identification unit 113 notifies a healthcare professional of an abnormal behavior.

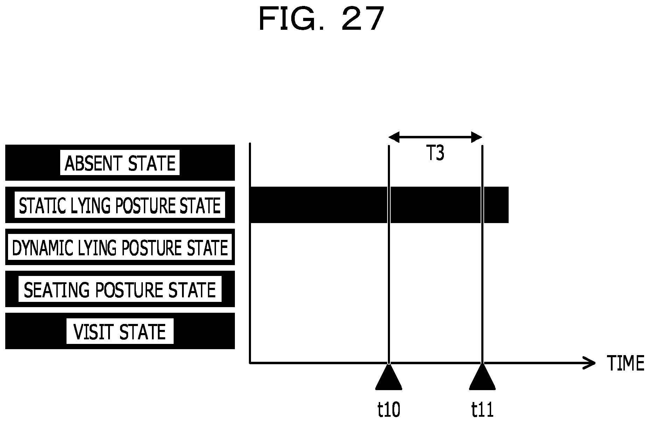

FIG. 27 illustrates an example of temporal change corresponding to a situation of not waking-up. When the state of the subject to be monitored 603 does not change from a static lying posture state even at time t11 after elapse of a period T3 from normal wake-up time t10, the situation identification unit 113 determines that the subject to be monitored 603 is in an abnormal situation. The normal wake-up time t10 is estimated from the past records. In this case, the situation identification unit 113 notifies a healthcare professional of a situation of not waking-up.

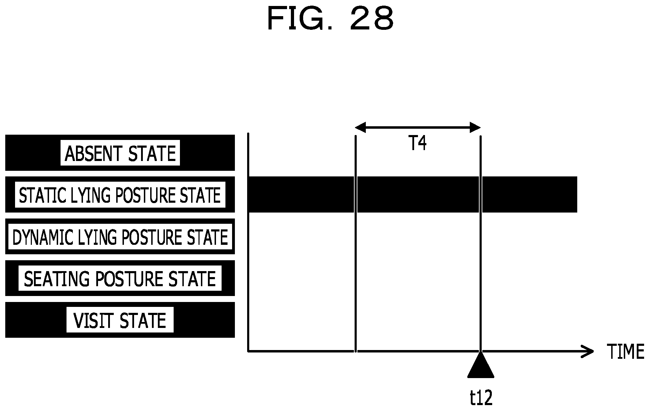

FIG. 28 illustrates an example of temporal change corresponding to a static lying posture state for a long time. When the state of the subject to be monitored 603 remains to be a static lying posture state at time t12 during a certain period T4 in the past, the situation identification unit 113 determines that it is highly probable that the subject to be monitored 603 has bedsores. In this case, the situation identification unit 113 notifies a healthcare professional of the static lying posture state for a long time.

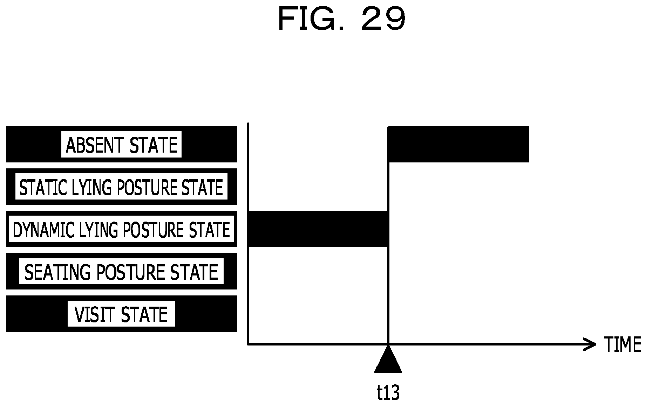

FIG. 29 illustrates an example of temporal change corresponding to falling off. When the state of the subject to be monitored 603 changes from a dynamic lying posture state to an absent state at time t13, the situation identification unit 113 determines that the subject to be monitored 603 is in a situation where the subject has fallen off the bed 601. In this case, the situation identification unit 113 notifies a healthcare professional of the falling off.

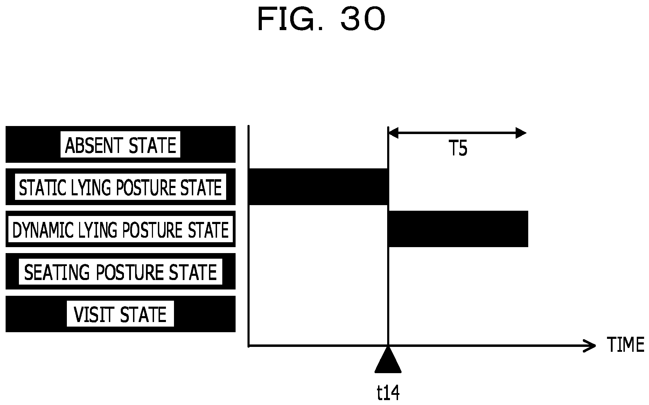

FIG. 30 illustrates an example of temporal change corresponding to a situation of being unable to get to sleep. When the state of the subject to be monitored 603 changes from a static lying posture state to a dynamic lying posture state at time t14, then a dynamic lying posture state continues for a period T5, the situation identification unit 113 determines that the subject to be monitored 603 is in a situation where the subject is unable to get to sleep. In this case, the situation identification unit 113 notifies a healthcare professional of the situation of being unable to get to sleep.

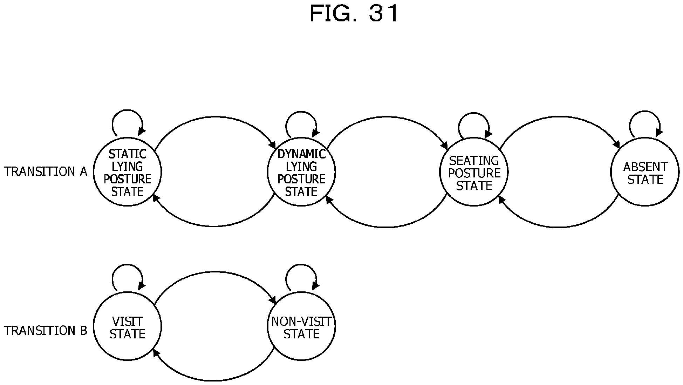

Next, the state change update processing that records a state change of the subject to be monitored 603, and the situation determination processing that determines a situation of the subject to be monitored 603 based on the recorded state change will be described with reference to FIGs. 31 to 44.

FIG. 31 illustrates examples of state transitions corresponding to state changes of the subject to be monitored 603. Transition A indicates a state transition in a non-visit state where a bed visitor is not detected. Transition B indicates a state transition for detection of a bed visitor.

In transition A, a possible transition destination from a static lying posture state is a static lying posture state or a dynamic lying posture state. A possible transition destination from a dynamic lying posture state is a static lying posture state, a dynamic lying posture state, or a seating posture state. A possible transition destination from a seating posture state is a dynamic lying posture state, a seating posture state, or an absent state. A possible transition destination from an absent state is a seating posture state or an absent state.

On the other hand, in transition B, a possible transition destination from a visit state where a bed visitor is detected is a visit state or a non-visit state. A possible transition destination from a non-visit state is a visit state or a non-visit state. In a non-visit state, the state of the subject to be monitored 603 is determined by a state transition in transition A.

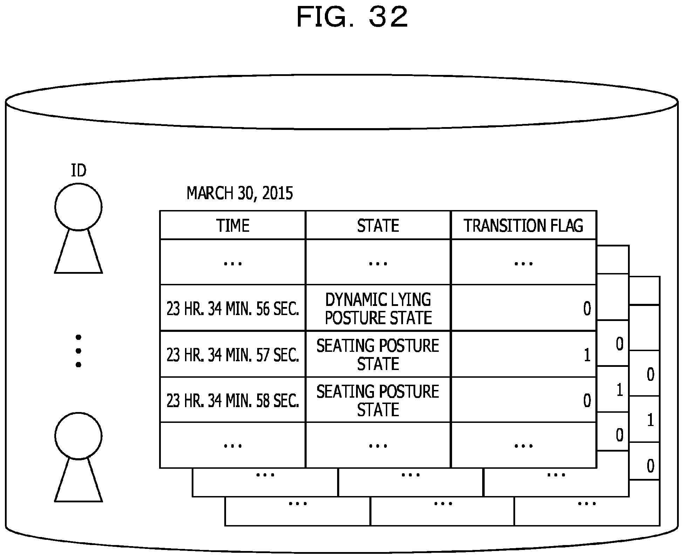

FIG. 32 illustrates an example of a result of detection of the state of the subject to be monitored 603 included in the state information 326. The result of detection includes a date, a time, a state, and a transition flag, and is recorded in association with ID of the subject to be monitored 603. The date and the time indicate a date and a time when the image 321 is captured. The state indicates a state that is detected from the image 321 by the state detection processing of FIG. 13. The transition flag has logical "1" when a detected state is different from the state at the previous time, and logical "0" when a detected state is the same as the state at the previous time.

Using those results of detection, the state detection unit 112 is able to generate a graph that indicates the temporal change illustrated in FIGs. 18 to 30, and to display the generated graph on a screen.

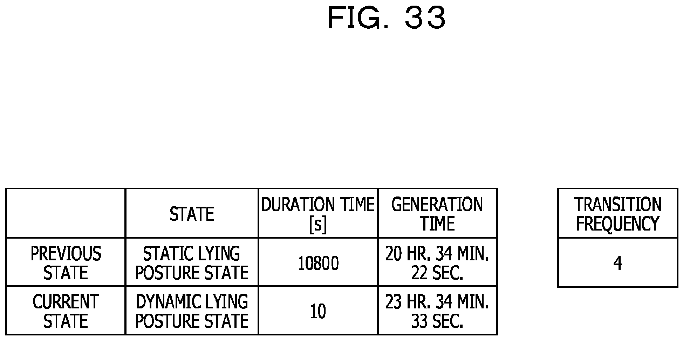

FIG. 33 illustrates examples of the previous state, the current state, and the transition frequency included in the state information 326. The current state corresponds to the state detected from the latest image 321. The previous state corresponds to the state detected from the image 321 one time unit before the latest image 321. The current state and the previous state each include a state, a duration time, and a generation time. The duration time indicates a time during which the state continues since an occurrence of the state. The generation time indicates a time when the state is generated. The transition frequency indicates the total of the values of transition flags in the results of detection included in the latest predetermined period, and corresponds to the number of state changes in the predetermined period.

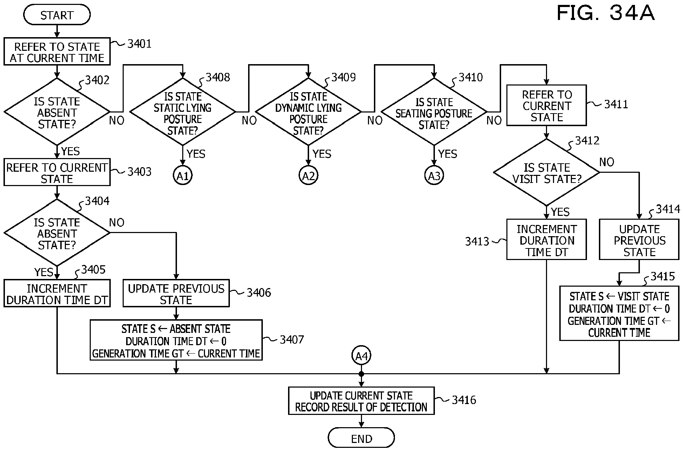

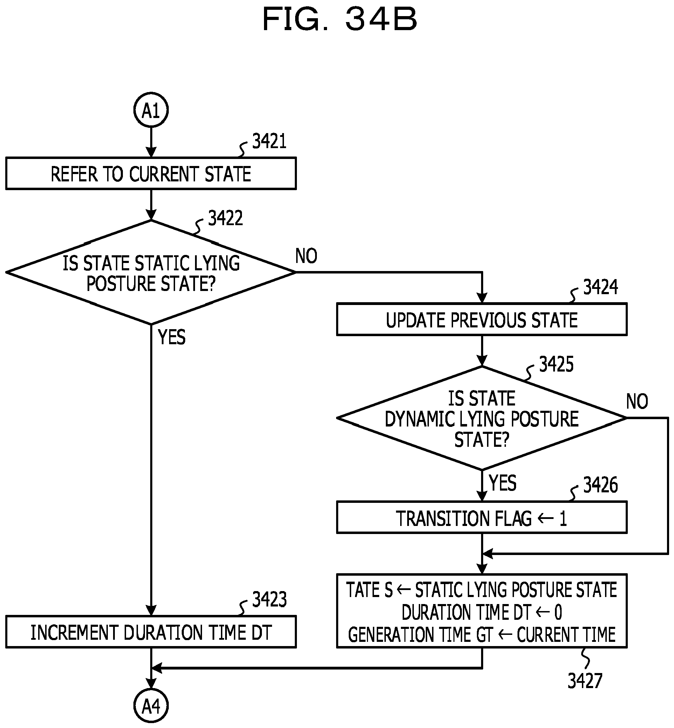

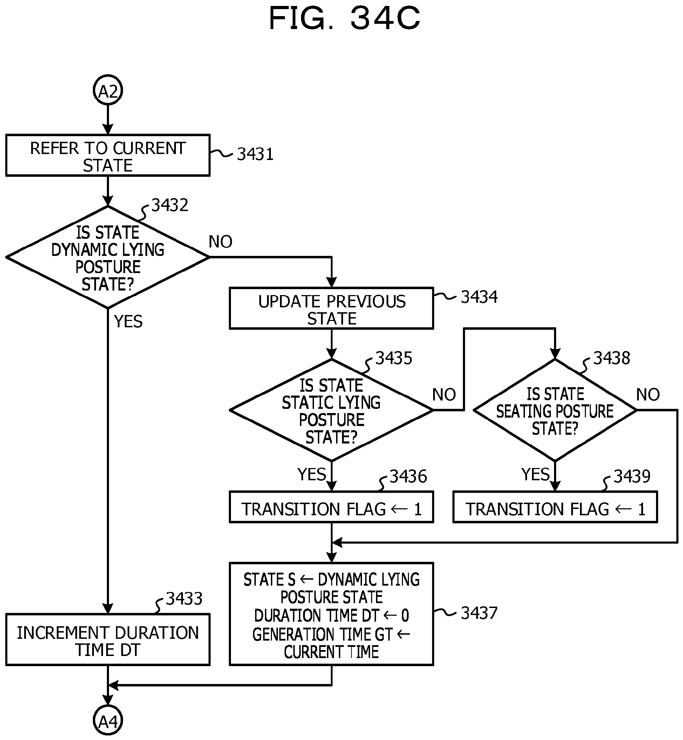

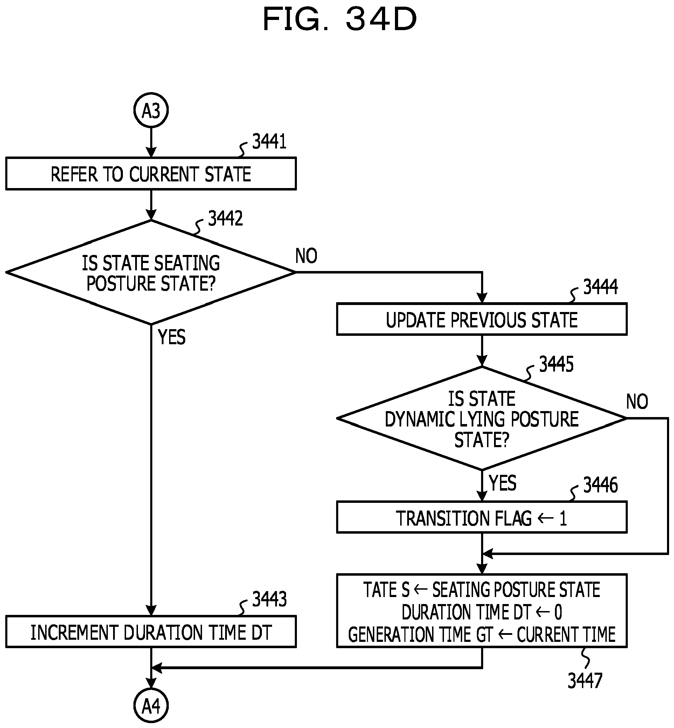

FIGs. 34A, 34B, 34C, and 34D are flowcharts illustrating examples of the state change update processing performed by the state detection unit 112 in the operation 1302, the operation 1310, the operation 1311, the operation 1313, and the operation 1314 of FIG. 13. In the state change update processing, a state S, a duration time DT, a generation time GT, and a transition flag F are used as parameters. In the initial state, the state S is set to indefinite. The duration time DT and the generation time GT are set to 0. The transition flag F is set to logical "0".

First, the state detection unit 112 refers to a state detected from the image 321 at the current time (operation 3401). The state detection unit 112 then checks whether or not the state at the current time is an absent state (operation 3402).

When the state at the current time is an absent state (YES in the operation 3402), the state detection unit 112 refers to the current state included in the state information 326 (operation 3403) and checks whether or not the current state is an absent state (operation 3404). At this point, the current state included in the state information 326 indicates the state at the previous time one time unit before the current time, and not the state at the current time.

When the current state is an absent state (YES in the operation 3404), the state detection unit 112 copies the duration time included in the current state to the duration time DT, and increments the duration time DT by just one time unit (operation 3405). The state detection unit 112 updates the current state by overwriting the current state with the duration time DT, and adds the result of detection of the absent state at the current time to the state information 326 (operation 3416).

On the other hand, when the current state is not an absent state (NO in the operation 3404), the state detection unit 112 updates the previous state by overwriting the previous state with included in the state information 326 with the current state (operation 3406). Subsequently, the state detection unit 112 sets the state S to an absent state, sets the duration time DT to 0, and sets the generation time GT to the current time (operation 3407). The state detection unit 112 then updates the current state by overwriting the current state with the state S, the duration time DT, and the generation time GT. The state detection unit 112 then adds the result of detection of the absent state at the current time to the state information 326 (operation 3416).

When the state at the current time is not an absent state (NO in the operation 3402), the state detection unit 112 checks whether or not the state at the current time is a static lying posture state (operation 3408). When the state at the current time is a static lying posture state (YES in the operation 3408), the state detection unit 112 refers to the current state included in the state information 326 (operation 3421). The state detection unit 112 then checks whether or not the current state is a static lying posture state (operation 3422).

When the current state is a static lying posture state (YES in the operation 3422), the state detection unit 112 copies the duration time included in the current state to the duration time DT, and increments the duration time DT by just one time unit (operation 3423). The state detection unit 112 updates the current state by overwriting the current state with the duration time DT, and adds the result of detection of the static lying posture state at the current time to the state information 326 (operation 3416).

On the other hand, when the current state is not a static lying posture state (NO in the operation 3422), the state detection unit 112 updates the previous state by overwriting the previous state with included in the state information 326 with the current state (operation 3424). The state detection unit 112 checks whether or not the current state is a dynamic lying posture state (operation 3425).

When the state at the current time is a dynamic lying posture state (YES in the operation 3425), the state detection unit 112 sets the transition flag F to logical "1" (operation 3426). Subsequently, the state detection unit 112 sets the state S to a static lying posture state, sets the duration time DT to 0, and sets the generation time GT to the current time (operation 3427). The state detection unit 112 then updates the current state by overwriting the current state with the state S, the duration time DT, and the generation time GT. The state detection unit 112 then adds the result of detection of the static lying posture state at the current time to the state information 326 (operation 3416).

On the other hand, when the current state is not a dynamic lying posture state (NO in the operation 3425), the state detection unit 112 performs the processing of the operation 3427 and after.

When the state at the current time is not a static lying posture state (NO in the operation 3408), the state detection unit 112 checks whether or not the state at the current time is a dynamic lying posture state (operation 3409). When the state at the current time is a dynamic lying posture state (YES in the operation 3409), the state detection unit 112 refers to the current state included in the state information 326 (operation 3431). The state detection unit 112 then checks whether or not the current state is a dynamic lying posture state (operation 3432).

When the current state is a dynamic lying posture state (YES in the operation 3432), the state detection unit 112 copies the duration time included in the current state to the duration time DT, and increments the duration time DT by just one time unit (operation 3433). The state detection unit 112 then updates the current state by overwriting the current state with the duration time DT. The state detection unit 112 then adds the result of detection of the dynamic lying posture state at the current time to the state information 326 (operation 3416).

On the other hand, when the current state is not a dynamic lying posture state (NO in the operation 3432), the state detection unit 112 updates the previous state by overwriting the previous state with included in the state information 326 with the current state (operation 3434). The state detection unit 112 then checks whether or not the current state is a static lying posture state (operation 3435).

When the state at the current time is a static lying posture state (YES in the operation 3435), the state detection unit 112 sets the transition flag F to logical "1" (operation 3436). Subsequently, the state detection unit 112 sets the state S to a dynamic lying posture state, sets the duration time DT to 0, and sets the generation time GT to the current time (operation 3437). The state detection unit 112 then updates the current state by overwriting the current state with the state S, the duration time DT, and the generation time GT. The state detection unit 112 then adds the result of detection of the dynamic lying posture state at the current time to the state information 326 (operation 3416).

On the other hand, when the current state is not a static lying posture state (NO in the operation 3435), the state detection unit 112 checks whether or not the state at the current time is a seating posture state (operation 3438). When the current state is a seating position state (YES in the operation 3438), the state detection unit 112 sets the transition flag F to logical "1" (operation 3439), and performs the processing of the operation 3437 and after. On the other hand, when the current state is not a seating posture state (NO in the operation 3438), the state detection unit 112 performs the processing of the operation 3437 and after.

When the state at the current time is not a dynamic lying posture state (NO in the operation 3409), the state detection unit 112 checks whether or not the state at the current time is a seating posture state (operation 3410). When the state at the current time is a seating posture state (YES in the operation 3410), the state detection unit 112 refers to the current state included in the state information 326 (operation 3441) and checks whether or not the current state is a seating posture state (operation 3442).

When the current state is a seating posture state (YES in the operation 3442), the state detection unit 112 copies the duration time included in the current state to the duration time DT, and increments the duration time DT by just one time unit (operation 3443). The state detection unit 112 then updates the current state by overwriting the current state with the duration time DT. The state detection unit 112 then adds the result of detection of the seating posture state at the current time to the state information 326 (operation 3416).

On the other hand, when the current state is not a seating posture state (NO in the operation 3442), the state detection unit 112 updates the previous state by overwriting the previous state with included in the state information 326 with the current state (operation 3444). The state detection unit 112 then checks whether or not the current state is a dynamic lying posture state (operation 3445).

When the state at the current time is a dynamic lying posture state (YES in the operation 3445), the state detection unit 112 sets the transition flag F to logical "1" (operation 3446). Subsequently, the state detection unit 112 sets the state S to a seating posture state, sets the duration time DT to 0, and sets the generation time GT to the current time (operation 3447). The state detection unit 112 then updates the current state by overwriting the current state with the state S, the duration time DT, and the generation time GT. The state detection unit 112 then adds the result of detection of the seating posture state at the current time to the state information 326 (operation 3416).

On the other hand, when the current state is not a dynamic lying posture state (NO in the operation 3445), the state detection unit 112 performs the processing of the operation 3447 and after.

When the state at the current time is not a seating posture state (NO in the operation 3410), the state at the current time is a visit state. Thus, the state detection unit 112 refers to the current state included in the state information 326 (operation 3411). The state detection unit 112 then checks whether or not the current state is a visit state (operation 3412).

When the current state is a visit state (YES in the operation 3412), the state detection unit 112 copies the duration time included in the current state to the duration time DT, and increments the duration time DT by just one time unit (operation 3413). The state detection unit 112 then updates the current state by overwriting the current state with the duration time DT. The state detection unit 112 then adds the result of detection of the visit state at the current time to the state information 326 (operation 3416).

On the other hand, when the current state is not a visit state (NO in the operation 3412), the state detection unit 112 updates the previous state by overwriting the previous state with included in the state information 326 with the current state (operation 3414). Subsequently, the state detection unit 112 sets the state S to a visit state, sets the duration time DT to 0, and sets the generation time GT to the current time (operation 3415). The state detection unit 112 then updates the current state by overwriting the current state with the state S, the duration time DT, and the generation time GT. The state detection unit 112 then adds the result of detection of the visit state at the current time to the state information 326 (operation 3416).

With this state change update processing, the previous state and the current state of FIG. 33 are updated according to a state detected from the image 321 at the current time. When the previous state is different from the current state, the previous state indicates the state before change, and the current state indicates the state after the change.

The situation identification unit 113 is able to identify the situation of the subject to be monitored 603 using the state, the current state, and the transition frequency of FIG. 33 in accordance with a situation identification rule. The situation identification rule is pre-stored in the memory unit 314.

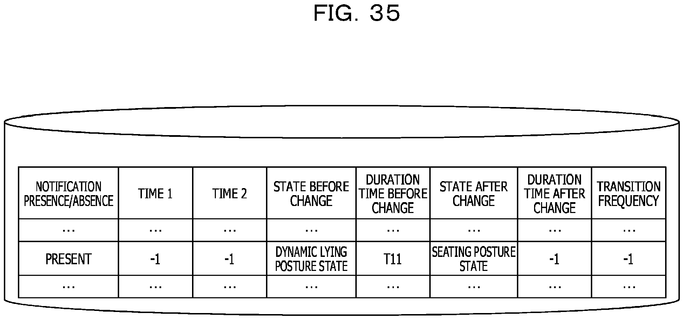

FIG. 35 illustrates a situation identification rule for a change from a dynamic lying posture state to a seating posture state. The situation identification rule of FIG. 35 includes items: notification presence/absence, time 1, time 2, state before change, duration time before change, state after change, duration time after change, and transition frequency.

The notification presence/absence is a parameter that designates whether or not a healthcare professional is to be notified of a situation. The time 1 and the time 2 are parameters that each designate a time used for identifying a situation. The state before change is a parameter that, when a state change occurs, designates a state before the change. The duration time before change is a parameter that designates a duration time of a state before change. The state after change is a parameter that, when a state change occurs, designates a state after the change. The duration time after change is a parameter that designates a duration time of a state after change. The transition frequency is a parameter that designates a threshold value for transition frequency.

In this example, the notification presence/absence is set to presence, the state before change is set to the dynamic lying posture state, the duration time before change is set to T11, and the state after change is set to the seating posture state. The time 1, the time 2, the duration time after change, and the transition frequency are set to -1 (don't care). The item set with -1 is not used as a condition that identifies a situation.

The situation identification rule of FIG. 35 indicates that when a dynamic lying posture state continues for the period T11 or longer, then the state changes from a dynamic lying posture state to a seating posture state, the situation of the subject to be monitored 603 is determined to be getting up, and a healthcare professional is notified of the situation. For instance, the temporal change illustrated in FIG. 19 matches the condition of the above-described situation identification rule. When the subject to be monitored 603 wakes up, a situation corresponding to getting up occurs. As the period T11, for instance, tens seconds to several minutes may be used.

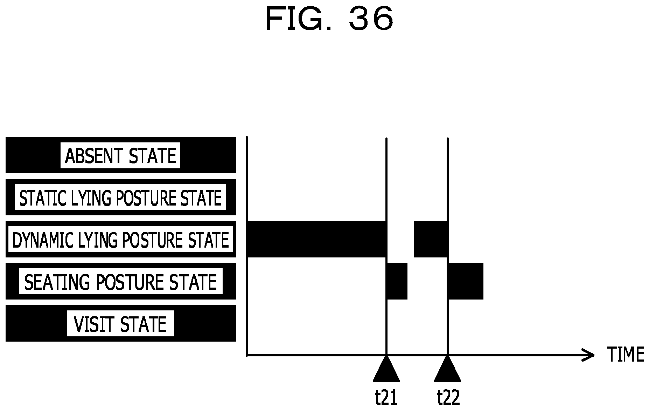

FIG. 36 illustrates an example of another change from a dynamic lying posture state to a seating posture state. When the state of the subject to be monitored 603 changes from a dynamic lying posture state to a seating posture state at time t21, and the duration time of the dynamic lying posture state before the change is the period T11 or longer, the situation of the subject to be monitored 603 is determined to be getting up, and a healthcare professional is notified of the situation.

Subsequently, the state of the subject to be monitored 603 changes from a seating posture state to a dynamic lying posture state and again changes from a dynamic lying posture state to a seating posture state at time t22. In this case, since the duration time of a dynamic lying posture state before the change is less than the period T11, the situation identification unit 113 does not determine that the situation of the subject to be monitored 603 is getting up, and a healthcare professional is not notified of the situation.

According to the situation identification rule of FIG. 35, when the subject to be monitored 603 wakes up, it is possible to notify a healthcare professional of getting up.

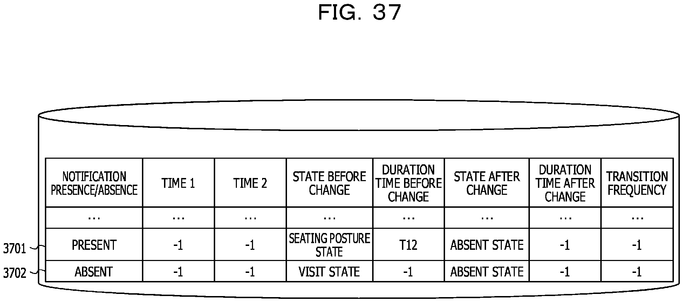

FIG. 37 illustrates an example of the situation identification rule for a change from a seating posture state or a visit state to an absent state. In a situation identification rule 3701, the notification presence/absence is set to presence, the state before change is set to the seating posture state, the duration time before change is set to T12, and the state after change is set to the absent state. The time 1, the time 2, the duration time after change, and the transition frequency are to -1.

The situation identification rule 3701 indicates that when a seating posture state continues for the period T12 or longer, then the state changes from a seating posture state to an absent state, the situation of the subject to be monitored 603 is determined to be leaving a bed alone, and a healthcare professional is notified of the situation. For instance, the temporal change illustrated in FIG. 20 matches the condition of the above-described situation identification rule 3701.

In contrast, in a situation identification rule 3702, the notification presence/absence is set to absence, the state before change is set to the visit state, and the state after change is set to the absent state. The time 1, the time 2, the duration time before change, the duration time after change, and the transition frequency are set to -1.

The situation identification rule 3702 indicates that when the state changes from a visit state to an absent state, the situation of the subject to be monitored 603 is determined to be leaving a bed along with the bed visitor 606, and a healthcare professional is not notified of the situation. For instance, the temporal change illustrated in FIG. 21 matches the condition of the above-described situation identification rule 3702.

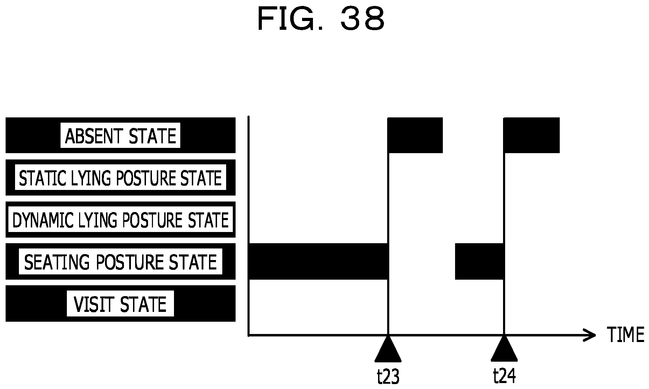

FIG. 38 illustrates an example of another change from a seating posture state to an absent state. When the state of the subject to be monitored 603 changes from a seating posture state to an absent state at time t23, and the duration time of the seating posture state before the change is the period T12 or longer, the situation of the subject to be monitored 603 is determined to be leaving a bed alone, and a healthcare professional is notified of the situation.

Subsequently, the state of the subject to be monitored 603 changes from an absent state to a seating posture state and again changes from a seating posture state to an absent state at time t24. In this case, since the duration time of a seating posture state before the change is less than the period T12, the situation identification unit 113 does not determine that the situation of the subject to be monitored 603 is leaving a bed alone, and a healthcare professional is not notified of the situation. For instance, a situation where after leaving the bed 601, the subject to be monitored 603 staggers and sits on the bed 601 again corresponds to the state change at time t24.

According to the situation identification rule 3701 of FIG. 37, when the subject to be monitored 603 leaves the bed 601 alone, it is possible to notify a healthcare professional of the leaving the bed alone.

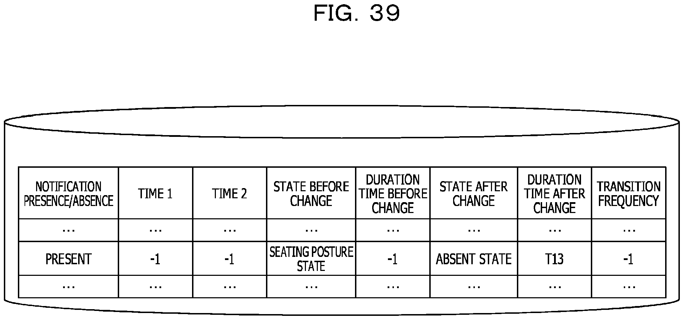

FIG. 39 illustrates an example of the situation identification rule for a change from a seating posture state to an absent state. In the situation identification rule of FIG. 39, the notification presence/absence is set to presence, the state before change is set to the seating posture state, the state after change is set to the absent state, and the duration time after change is set to T13. The time 1, the time 2, the duration time before change, and the transition frequency are set to -1.

The situation identification rule of FIG. 39 indicates that when the state changes from a seating posture state to an absent state, then an absent state continues for the period T13 or longer, the situation of the subject to be monitored 603 is determined to be leaving the bed alone for a long time, and a healthcare professional is notified of the situation. For instance, the temporal change illustrated in FIG. 20 matches the condition of the above-described situation identification rule. In this case, a situation may have occurred where after the subject to be monitored 603 falls down, the subject does not get up for a long time, or the subject to be monitored 603 wanders around.

According to the situation identification rule of FIG. 39, when the subject to be monitored 603 falls down or wanders around, it is possible to notify a healthcare professional of leaving the bed alone for a long time.

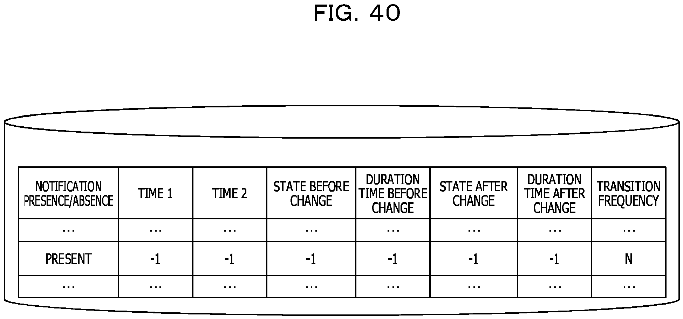

FIG. 40 illustrates an example of the situation identification rule for transition frequency. In the situation identification rule of FIG. 40, the notification presence/absence is set to presence, and the transition frequency is set to N (an integer of 2 or greater). The time 1, the time 2, the state before change, the duration time before change, the state after change, and the duration time after change are set to -1.

The situation identification rule of FIG. 40 indicates that when the number of state changes which have occurred in the latest predetermined period between a static lying posture state, a dynamic lying posture state, a seating posture state, and an absent state is greater than N, the situation of the subject to be monitored 603 is determined to be abnormal, and a healthcare professional is notified of the situation. For instance, the temporal change illustrated in FIG. 25 matches the condition of the above-described situation identification rule. In this case, a situation may have occurred where the subject to be monitored 603 is struggling or the subject to be monitored 603 is acting violently.

According to the situation identification rule of FIG. 40, when the subject to be monitored 603 is struggling or acting violently, on behalf of the subject to be monitored 603, it is possible to notify a healthcare professional of the abnormal behavior.

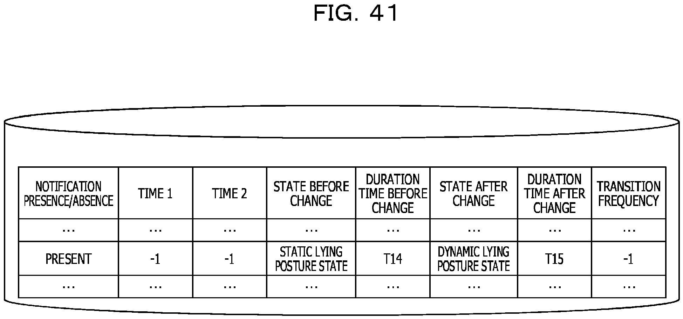

FIG. 41 illustrates an example of the situation identification rule for a change from a static lying posture state to a dynamic lying posture state. In the situation identification rule of FIG. 41, the notification presence/absence is set to presence, the state before change is set to the static lying posture state, the duration time before change is set to T14, the state after change is set to the dynamic lying posture state, and the duration time after change is set to T15. The time 1, the time 2, and the transition frequency are set to -1.

The situation identification rule of FIG. 41 indicates that when a static lying posture state continues for the period T14 or longer, then the state changes from a static lying posture state to a dynamic lying posture state, and a dynamic lying posture state continues for the period T15 or longer, the situation of the subject to be monitored 603 is determined to be waking-up or starting to move, and a healthcare professional is notified of the situation. For instance, the temporal change illustrated in FIG. 18 matches the condition of the above-described situation identification rule.

In contrast, in the case of the temporal change illustrated in FIG. 22, the duration time of a dynamic lying posture state is less than the period T15, and thus the situation identification unit 113 does not determine that the situation is waking-up or starting to get up, and a healthcare professional is not notified of the situation. Therefore, notification of mere rolling over of the subject to be monitored 603 is avoided.

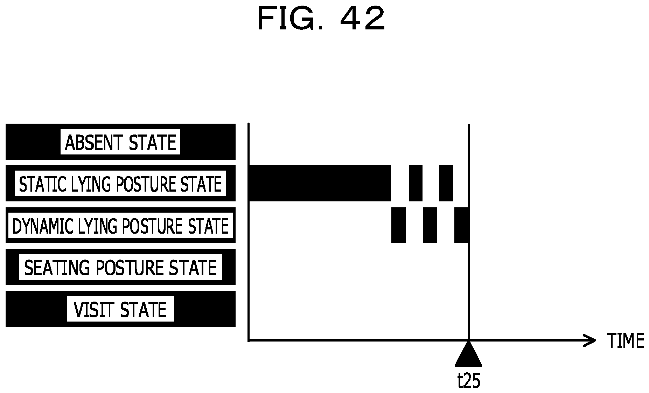

FIG. 42 illustrates an example of another change from a static lying posture state to a dynamic lying posture state. In this example, a state change from a static lying posture state to a dynamic lying posture state occurs multiple times. However, the duration time of each dynamic lying posture state is less than the period T15, and thus the situation identification unit 113 does not determine that the situation is waking-up or starting to get up.

However, when the number of state changes which have occurred in the latest predetermined period is greater than N at time t25, this matches the condition of the situation identification rule of FIG. 40, and thus the situation identification unit 113 determines that the situation of the subject to be monitored 603 is abnormal, and a healthcare professional is notified of the situation.

According to the situation identification rule of FIG. 41, when the subject to be monitored 603 has woken up or started to get up, it is possible to notify a healthcare professional of the waking-up or starting to get up.

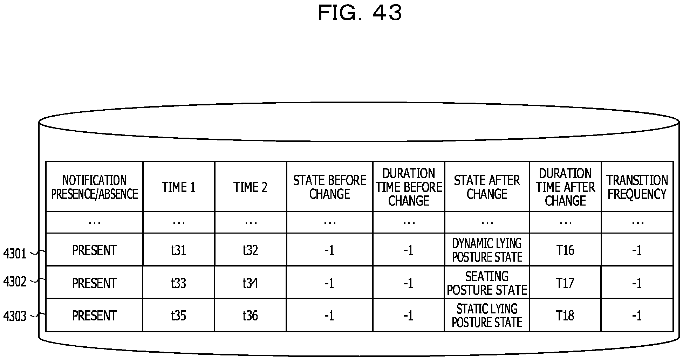

FIG. 43 illustrates an example of the situation identification rule for time. In a situation identification rule 4301, the notification presence/absence is set to presence, the time 1 is set to t31, the time 2 is set to t32, the state after change is set to the dynamic lying posture state, and the duration time after change is set to T16. The state before change, the duration time before change, and the transition frequency are set to -1.

The situation identification rule 4301 indicates that when a dynamic lying posture state occurs between time t31 and time t32, then continues for a period T16 or longer, the situation of the subject to be monitored 603 is determined to be a dynamic lying posture state for a long time, and a healthcare professional is notified of the situation. As time t31, for instance, a bedtime may be used, and as time t32, for instance, a wake-up time may be used. As the period T16, for instance, 10 minutes to one hour may be used.

In a situation identification rule 4302, the notification presence/absence is set to presence, the time 1 is set to t33, the time 2 is set to t34, the state after change is set to the seating posture state, and the duration time after change is set to T17. The state before change, the duration time before change, and the transition frequency are set to -1.

The situation identification rule 4302 indicates that when a seating posture state occurs between time t33 and time t34, then continues for a period T17 or longer, the situation of the subject to be monitored 603 is determined to be a seating posture state for a long time, and a healthcare professional is notified of the situation. As time t33, for instance, a bedtime may be used, and as time t34, for instance, a wake-up time may be used. As the period T17, for instance, 10 minutes to one hour may be used.

In a situation identification rule 4303, the notification presence/absence is set to presence, the time 1 is set to t35, the time 2 is set to t36, the state after change is set to the static lying posture state, and the duration time after change is set to T18. The state before change, the duration time before change, and the transition frequency are set to -1.

The situation identification rule 4303 indicates that when a static lying posture state occurs between time t35 and time t36, then continues for a period T18 or longer, the situation of the subject to be monitored 603 is determined to be a static lying posture state for a long time, and a healthcare professional is notified of the situation. For instance, the temporal change illustrated in FIG. 28 matches the condition of the above-described situation identification rule 4303. As the period T18, for instance, one hour to several hours may be used.

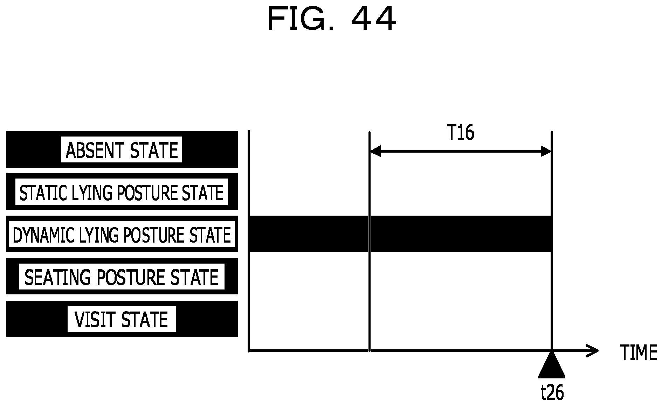

FIG. 44 illustrates an example of a dynamic lying posture state for a long time. When a dynamic lying posture state occurs between time t31 and time t32, then continues for a period T16 or longer at time t26, the situation of the subject to be monitored 603 is determined to be a dynamic lying posture state for a long time, and a healthcare professional is notified of the situation. In this case, a situation may have occurred where the subject to be monitored 603 is not sleeping.

On the other hand, when a dynamic lying posture state occurs at a time not between time t31 and time t32, then continues for a period T16 or longer, the situation identification unit 113 does not determine that the situation of the subject to be monitored 603 is a dynamic lying posture state for a long time, and a healthcare professional is not notified of the situation.

According to the situation identification rule of FIG. 43, when an expected behavior is not performed by the subject to be monitored 603 in a predetermined time range, a healthcare professional may be notified of the abnormal behavior.

The configuration of the situation identification device 101 of FIGs. 1 and 3 is merely an example, and part of the components may be omitted or changed according to an application purpose or conditions of the situation identification device 101. For instance, in the situation identification device 101 of FIG. 3, when the bed area detection processing and the head area detection processing are performed externally of the situation identification device 101, the image acquisition unit 311, the bed area detection unit 312, and the head area detection unit 313 may be omitted.

The flowcharts of FIGs. 2, 4, 9 to 11, 13, 14, 34A, 34B, 34C, and 34D are merely examples, and part of the processing may be omitted or changed according to the configuration or conditions of the situation identification device 101. For instance, in the situation identification processing of FIG. 4, when the bed area detection processing and the head area detection processing are performed externally of the situation identification device 101, the processing of the operations 401 to 403 may be omitted.

In the bed visitor detection processing of FIG. 9, the processing in either one of the operation 901 and the operation 902 may be omitted. The area LU may be used instead of the area LD in the operation 1003, the operation 1004, and the operation 1010 of the left-side detection processing of FIG. 10. Similarly, the area RU may be used instead of the area RD in the operation 1103, the operation 1104, and the operation 1110 of the right-side detection processing of FIG. 11.

In the state detection processing of FIG. 13, when a seating posture state is not included in the objects to be detected, the processing of the operation 1312 and the operation 1313 may be omitted. When the head area is not present in the lying posture area in the operation 1307, the state detection unit 112 performs the processing of the operation 1314.

When an absent state is not included in the objects to be detected, the processing of the operation 1312 and the operation 1314 may be omitted. When the head area is not present in the lying posture area in the operation 1307, the state detection unit 112 performs the processing of the operation 1313.

When a dynamic lying posture state and a static lying posture state are not distinguished, the processing of the operations 1308 to 1311 may be omitted. When the head area is present in the lying posture area in the operation 1307, the state detection unit 112 determines that the state of the subject to be monitored 603 is a lying posture state, and generates state information 326 that indicates a lying posture state.

When a dynamic lying posture state, a static lying posture state, and a seating posture state are not distinguished, the processing of the operations 1307 to 1313 may be omitted. After performing the processing of the operation 1306, the state detection unit 112 checks whether or not the head area is present in the first monitoring area. When the head area is present in the first monitoring area, the state detection unit 112 determines that the state of the subject to be monitored 603 is a present state. The state detection unit 112 then generates state information 326 that indicates a present state. On the other hand, when the head area is not present in the first monitoring area, the state detection unit 112 performs the operation 1314.

When the reliability of the head area is sufficiently high, the processing of the operations 1304 to 1306 may be omitted.

In the operation 1405 of the head area correction processing of FIG. 14, the state detection unit 112 may use a range narrower than the head area at the previous time or a range wider than the head area at the previous time as a predetermined range.

Also, in the operation 1404, the state detection unit 112 may calculate the distance between the position of the head area in the image 321 at the previous time, and the position of the head area in the image 321 at the current time as the amount of movement. In this case, in the operation 1405, the threshold value for distance is used as a predetermined range. When the distance is less than or equal to the threshold value, the state detection unit 112 determines that the amount of movement is in the predetermined range. On the other hand, when the distance is larger than the threshold value, the amount of movement is determined to be out of the predetermined range.

In the state change update processing of FIGs. 34A, 34B, 34C, and 34D, when a seating posture state or an absent state is not included in the objects to be detected, determination regarding a seating posture state or an absent state may be omitted. When a dynamic lying posture state and a static lying posture state are not distinguished, determination regarding a dynamic lying posture state and a static lying posture state may be changed to determination regarding a seating posture state.

The first monitoring area, the lying posture area and the seating posture area of FIGs. 5 and 7 are merely examples, and an area in a different shape or position may be used. The second monitoring areas of FIGs. 6 to 8 are merely examples, and an area in a different shape or position may be used. Instead of dividing the second monitoring area into five areas, the second monitoring area may be divided into a different number of areas. The area LU and the area LD may be integrated to an area, and the area RU and the area RD may be integrated to an area. As the detection area 801 of FIG. 8, an area in a different shape or position may be used. For instance, the entirety of the areas of the area LU, the area LD, the area D, the area RD and the area RU may be used as the detection area 801.

The XYZ coordinate system of FIGs. 6 and 12 is merely an example, and another three-dimensional coordinate system may be used. The position of the imaging device 301 of FIG. 12 is merely an example, and the imaging device 301 may be disposed at a different position. Also, the shape of the bed 601 is merely an example, and the bed 601 in a different shape may be used.

The head area correction processing of FIG. 16C is merely an example, and the head area at the previous time and the head area at the current time change according to the image 321. An area in a different shape may be used as the head area.

The temporal changes in the state information 326 of FIGs. 17 to 30, 36, 38, 42, and 44 are merely examples, and the state information 326 changes according to the image 321. The state transition of FIG. 31 is merely an example, and a state transition including another state of the subject to be monitored 603 may be used. The results of detection of FIG. 32 and the previous state, the current state, and the transition frequency of FIG. 33 are merely examples, and the state information 326 in another format may be used. The situation identification rules of FIGs. 35, 37, 39, 40, 41, and 43 are merely examples, and another situation identification rule may be used.

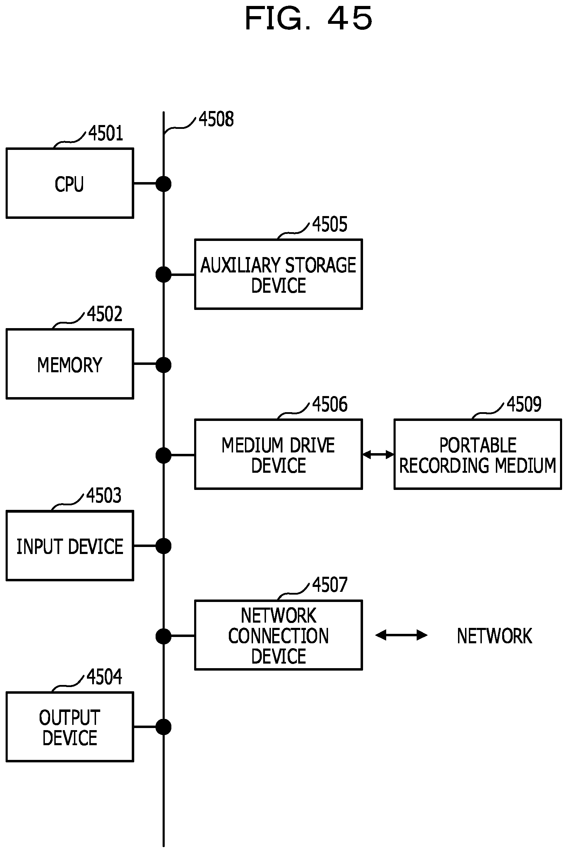

FIG. 45 illustrates a configuration example of an information processing device (computer) used as the situation identification device 101 of FIGs. 1 and 3. The information processing device of FIG. 45 includes a central processing unit (CPU) 4501, a memory 4502, an input device 4503, an output device 4504, an auxiliary storage device 4505, a medium drive device 4506, and a network connection device 4507. These components are connected to each other by a bus 4508. The imaging device 301 of FIG. 3 may be connected to the bus 4508.

The memory 4502 is a semiconductor memory such as a read only memory (ROM), a random access memory (RAM), or a flash memory, for instance, and stores programs and data which are used for situation identification processing. The memory 4502 may be used as the memory unit 314 of FIG. 3.

The CPU 4501 (processor) executes a program utilizing the memory 4502, for instance, and thereby operates as the area identification unit 111, the state detection unit 112, and the situation identification unit 113 of FIGs. 1 and 3. The CPU 4501 executes a program utilizing the memory 4502, and thereby operates also as the image acquisition unit 311, the bed area detection unit 312, and the head area detection unit 313 of FIG. 3.

The input device 4503 is, for instance, a keyboard or a pointing device, and is used for input of directions or information from an operator or a user. The output device 4504 is, for instance, a display device, a printer or a speaker, and is used for an inquiry to an operator or a user, or output of processing results. The processing results may be the state information 326 or the situation information 327. The output device 4504 may be used as the output unit 114 of FIGs. 1 and 3.

The auxiliary storage device 4505 is, for instance, a magnetic disk drive, an optical disk drive, a magnetic optical disk drive, a tape drive. The auxiliary storage device 4505 may be a hard disk drive. The information processing device may store programs and data in the auxiliary storage device 4505, and may use the programs and data by loading them into the memory 4502. The auxiliary storage device 4505 may be used as the memory unit 314 of FIG. 3.

The medium drive device 4506 drives a portable recording medium 4509, and accesses contents recorded. The portable recording medium 4509 is a memory device, a flexible disk, an optical disk, or a magnetic optical disk, etc. The portable recording medium 4509 may be a compact disk read only memory (CD-ROM), a digital versatile disk (DVD), or a universal serial bus (USB) memory, etc. An operator or a user may store programs and data in the portable recording medium 4509, and may use the programs and data by loading them into the memory 4502.

Like this, a computer-readable recording medium that stores programs and data used for the situation identification processing is a physical (non-transitory) recording medium such as the memory 4502, the auxiliary storage device 4505, or the portable recording medium 4509.

The network connection device 4507 is a communication interface that is connected to communication network such as Local Area Network, Wide Area Network, and that performs data conversion accompanying communication. The information processing device may receive programs and data from an external device via the network connection device 4507, and may use the programs and data by loading them into the memory 4502.

The information processing device may receive a processing request from a user terminal via the network connection device 4507, may perform the situation identification processing to transmit a result of the processing to a user terminal. In this case, the network connection device 4507 may be used as the output unit 114 of FIGs. 1 and 3.

The information processing device does not have to include all the components of FIG. 45, and part of the components may be omitted according to an application purpose or conditions. For instance, directions or information may not be inputted from an operator or a user, and the input device 4503 may be omitted. When the portable recording medium 4509 or a communication network is not utilized, the medium drive device 4506 or the network connection device 4507 may be omitted.

## Claims

1. A situation identification method executed by a processor included in a situation identification device, the situation identification method comprising:
acquiring a plurality of images;
identifying, for each of the plurality of images, a first area including a bed area where a place to sleep appears in an image, and a second area where an area in a predetermined range around the bed appears in the image;
detecting a state of a subject to be monitored for each of the plurality of images based on a result of detection of a head area indicating an area of a head of the subject to be monitored in the first area and a result of detection of a living object in the second area;
when the state of the subject to be monitored changes from a first state to a second state, identifying a situation of the subject to be monitored based on a combination of the first state and the second state; and
outputting information that indicates the identified situation.

2. The situation identification method according to claim 1, wherein
the result of detection of the head area indicates presence or absence of the head area in the first area, and
the result of detection of the living object indicates presence or absence of a dynamic area in the second area.

3. The situation identification method according to claim 1, wherein
the state of the subject to be monitored is one of a visit state indicating presence of a visitor to the place to sleep, a present state indicating that the subject to be monitored is present on the place to sleep, and an absent state indicating that the subject to be monitored is not present on the place to sleep, and
the detecting includes:
when the dynamic area is present in the second area, detecting the visit state;
when the head area is present in the first area, detecting the present state; and
when the head area is not present in the first area, detecting the absent state.

4. The situation identification method according to claim 3, wherein
the first area includes a lying posture area and a seating posture area, and
when the head area is present in the first area, the result of detection of the head area indicates whether the head area is present in the lying posture area or the seating posture area, and
the present state is one of a dynamic lying posture state indicating that the subject to be monitored is lying and moving on the bed, a static lying posture state indicating that the subject to be monitored is lying and not moving on the place to sleep, and a seating posture state indicating that the subject to be monitored is sitting on the place to sleep, and
the detecting includes:
when the head area is present in the lying posture area and the dynamic area is present in the first area, detecting the dynamic lying posture state;
when the head area is present in the lying posture area and the dynamic area is not present in the first area, detecting the static lying posture state; and
when the head area is present in the seating posture area, detecting the seating posture state.

5. The situation identification method according to claim 4,
wherein the identifying the situation includes determining that the situation of the subject to be monitored is that the subject to be monitored has woken up on the place to sleep when the first state is the dynamic lying posture state and the second state is the seating posture state.

6. The situation identification method according to claim 4,
wherein the identifying the situation includes determining that the situation of the subject to be monitored is that the subject to be monitored has left the place to sleep alone when the first state is the seating posture state and the second state is the absent state.

7. The situation identification method according to claim 4,
wherein the identifying the situation includes determining that the situation of the subject to be monitored is that the subject to be monitored has left the place to sleep along with the visitor when the first state is the visit state and the second state is the absent state.

8. The situation identification method according to claim 4, wherein the detecting the state includes detecting a plurality of states including the first state and the second state from each of the plurality of images captured in a predetermined time period, and
the identifying the situation includes determining that the situation of the subject to be monitored is abnormal behavior, when the detected plurality of states include the dynamic lying posture state, the static lying posture state and the seating posture state, and the number of state changes in the plurality of states is greater than a predetermined number.

9. The situation identification method according to any of claims 1 to 8, wherein the detecting the state includes:
when a relative position of a second head area in the first area contained in a second image captured at a second time with respect to a first head area in the first area contained in a first image captured at a first time before the second time is out of a predetermined range and at least one of the first head area and the second head area is not the dynamic area, determining that the second head area is erroneously detected; and
replacing a result of detection of the second head area at the second time by a result of detection of the first head area at the first time.

10. The situation identification method according to any of claims 1 to 8, wherein the detecting the state includes:
when the head area is present in the first area contained in a first image captured at a first time and the head area is not present in the first area contained in a second image captured at a second time after the first time, determining that the head area has not been detected at the second time; and
using a result of detection of the head area at the first time as a result of detection of the head area at the second time.

11. A situation identification device comprising:
an image acquisition unit that acquires a plurality of images;
an area identification unit that identifies, for each of the plurality of images, a first area including a bed area where a place to sleep appears in an image, and a second area where an area in a predetermined range around the place to sleep appears in the image;
a state detection unit that detects a state of a subject to be monitored for each of the plurality of images based on a result of detection of a head area indicating an area of a head of the subject to be monitored in the first area and a result of detection of a living object in the second area;
a situation identification unit that identifies, when the state of the subject to be monitored changes from a first state to a second state, a situation of the subject to be monitored based on a combination of the first state and the second state; and
an output unit that outputs information that indicates the identified situation.

12. A situation identification program that causes a processor included in a situation identification device to execute a process, the process comprising:
acquiring a plurality of images;
identifying, for each of the plurality of images, a first area including a bed area where a place to sleep appears in an image, and a second area where an area in a predetermined range around the place to sleep appears in the image;
detecting a state of a subject to be monitored for each of the plurality of images based on a result of detection of a head area indicating an area of a head of the subject to be monitored in the first area and a result of detection of a living object in the second area;
when the state of the subject to be monitored changes from a first state to a second state, identifying a situation of the subject to be monitored based on a combination of the first state and the second state; and
outputting information that indicates the identified situation.
